# EUROPEAN PATENT APPLICATION

(11) **EP 2 583 962 A2**
(43) Date of publication of application: **24.04.2013**
(21) Application number: 11796003.9
(22) Date of filing: 17.06.2011
(51) Int. Cl.: C07C 335/08, C07C 275/18, A61K 31/17, A61P 31/18

(54) **NOVEL THIOUREA OR UREA DERIVATIVE, PREPARATION METHOD THEREOF, AND PHARMACEUTICAL COMPOSITION FOR PREVENTING OR TREATING AIDS, CONTAINING SAME AS ACTIVE INGREDIENT**

(30) Priority: 18.06.2010 KR 20100057970
(71) Applicant: Avixgen Inc., Seoul 137-040 (KR)
(72) Inventor: YOU, Ji Chang, Seoul 130-070 (KR); HAN, Gyoon Hee, Hwaseong-si Gyeonggi-do 445-982 (KR); LEE, Chul Ho, Seoul 157-904 (KR); SONG, Doo Na, Seoul 133-020 (KR); CHUNG, Koo Hwan, Seoul 135-270 (KR)
(74) Representative: Towler, Philip Dean
(86) International application number: PCT/KR2011/004459
(87) International publication number: WO 2011/159137

(57) **Abstract**

Disclosed are novel thiourea or urea derivatives inhibitory of HIV activity. Also provided are a method for preparing the thiourea or urea derivatives, and a pharmaceutical composition for the prophylaxis or therapy of AIDS comprising the derivatives. Having high inhibitory activity against HIV, the thiourea or urea derivatives can be effectively used in the prophylaxis or therapy of AIDS.

## Description

### Technical Field

The present invention relates to a novel thiourea or urea derivative, a method for preparing the same, and a pharmaceutical composition for the prevention or treatment of AIDS, comprising the same as an active ingredient.

### Background Art

AIDS was first clinically observed in 1981, and HIV (Human Immunodeficiency Virus) was discovered as causing AIDS as it was isolated from AIDS patients in 1984.

Taxonomically, HIV is a member of the genus *Lentivirus,* part of the family of Retroviridae. An HIV particle is roughly spherical with a diameter of about 10 microns. Its genome is composed of two copies of RNA enclosed by a protein sac (capsid, core protein) which is, in turn, surrounded by a viral envelope composed of phospholipids, like general plasma membranes. The HIV genome codes for 10 genes, which are too many relative to the total size of the genome.

HIV infection is mediated through interaction of the envelope glycoproteins (gp120) on the viral surface with receptors on the target cell. The cell surface protein molecules that act as the receptors are mainly CD4 antigens. This is why cells that express CD4 on their surface (CD4+ cells), such as macrophages and helper T cells, are the main target cells of HIV. Following the adsorption of the glycoprotein to the receptors, the viral phospholipid envelope is fused with the cell membrane with the concomitant release of the HIV genome and nucleocapsid into the cell. Upon entry into the target cell, the viral RNA genome is converted into DNA by a virally encoded reverse transcriptase that is transported along with the viral genome in the virus particle. The viral DNA is then transported into the cell nucleus and integrated into the host cell's genome. This procedure is one of the unique features that retroviruses have. As such, the integrated viral DNA lies dormant in the safest site of the host cell while being supplied with all mechanisms and resources necessary for the growth of the virus from the cell. Further, HIV protects itself from and survives the immune system while proliferating or entering the latent stage of infection depending on surrounding situations and conditions.

Two types of AIDS viruses are characterized: HIV-1 and HIV-2. HIV-1 is found in patients all over the world and is the cause of the majority of HIV infections globally. HIV-2 is largely confined to West Africa. Its nucleotide sequence is only 55 % identical to that of HIV-1, and rather more similar to that of SIV (Simian Immunodeficiency Virus), known as monkey AIDS virus. HIV-2 is less virulent than HIV-1. HIV not only has very high biological variability, but also very high genetic variability. The nucleotide sequences of HIV differ from one AIDS patient to another, and vary with the progress of AIDS even in the same patient. What is more serious, the nucleotide sequences of HIV, when sampled at the same time from the same patient, are different depending on sampling tissue. These HIV sequence polymorphisms are strongly associated with various biological characteristics of the virus. Given different nucleotide sequences, HIV differs in target cell preference for its infection, virion productivity, cytotoxicity, ability to form multinucleated giant cells, latent period and active period, and sensitivity to neutralizing antibodies. More recent studies on the relationship between variable biological properties of HIV and pathogenesis of HIV infection show that most AIDS viruses isolated from patients in the early stages do not create multinucleated giant cells (nonsyncytia-inducing (NSI)) and prefer macrophages for their infection, whereas HIV is increasingly liable to create multinucleated giant cells (syncytia-inducing (SI)), and replicate preferably in T-helper cells, with the progression of AIDS, indicating that there is strong relationship between biological properties of HIV and pathogenesis of HIV infection.

One week after HIV infection, the virus actively proliferates and can be easily detected in the blood of the infectee, that is, he or she becomes viremic. Then, the virus rapidly decreases to such a low level within one to two weeks that it cannot be isolated. After maintenance of such latency for a significant period of time, HIV actively replicates with the onset of AIDS, so that viremia occurs. Recent PCR research has attracted attention because of its report that HIV replicates while it is dormant. After HIV infection, the level of CD4 cells rapidly decreases during the primary viremia period, and then is recovered to a constant value (healthy person: 500-1000 CD4 cells/mm²). Since that point, the blood level of CD4 cells gradually decreases over several years. When CD4 cells drop below 200 counts per mm³ of blood, the onset of ARC (AIDS-related complex) or AIDS takes place. AIDS patients experience a condition in which progressive failure of the immune system allows life-threatening opportunistic infections, such as *Pneumocysitis carinii* pneumonia, to thrive. The time period during which the HIV level rapidly reduces after the primary viremia is incident with the activation period of CD8 cells. CD8 T cells are known to act to inhibit cell growth or selectively kill virus-infected cells. Hence, CD8 T cells seem to account for immunity against the early infection virus. Antibodies are produced after the virus level is reduced. CD8 cells and antibodies continue to exist during the time period from the initial time of infection to the onset of AIDS, but their functions have been reduced, or denatured, and they even promote the HIV infection. How the immune system that exhibits apparent anti-viral activity in the early stage of infection is lost remains a problem that is yet to be solved. Due to AIDS specificity for humans, the understanding of the etiology of HIV is at an extremely low level. Although there is consensus among scientists about the fact that a reduction in CD4 cell level is a direct cause of immunodeficiency, there are various different opinions on how HIV reduces the level of CD4 cells. Suggested theories accounting for the reduction of CD4 cell level include the formation of multinucleated giant cells, the accumulation of non-inserted viral DNA, influence on the structure of host cell membranes, the induction of programmed cell death, the secretion of toxic matter from infected cells, and autoimmune-mediated cytolysis. None of them, however, have thus far been proven as fact, or have been shown to occur in practice *in vivo.* Extensive research into HIV pathogenesis has been conducted on monkeys using the monkey AIDS virus SIV, but it has yielded no novel findings.

Most prevalent among currently available AIDS drugs is AZT (zidovudine), which is an HIV reverse transcriptase inhibitor. It remains in widespread use today and is recognized as one of the most effective drugs in medical history. This drug is clinically very effective in the early stages of HIV infection, but is found to have no positive influences on the extension of a patient's life because of its side effects. That is, AZT induces bone marrow toxicity, and may allow HIV to become AZT-resistant over time. DDI, DDC, and d4T, all functioning like AZT, and approved by the FDA, are found to be less toxic than AZT, but induce HIV to be resistant thereto.

There are many anti-viral methods that have been developed that are awaiting efficacy testing. Examples of them are the prevention of viral adsorption to cells, the selective killing of virus-infected cells, the use of inhibitors against enzymes playing an important role in viral growth (e.g., protease, integrase, tat inhibitor, rev inhibitor, etc.), cytokine therapies, injection of CD8 cells, and gene therapies. There has also been a great advance in the development of HIV vaccines, such as the use of a dead virus, or attenuated virus, which is alive but not pathogenic, an isolated viral protein expressed by bioengineering (subunit vaccine), an anti-idiotypic antibody, and the direct injection of a DNA gene. However, a fundamental problem with the development of vaccines lies in the excessive diversity of HIV viruses. For example, a person immunized with a vaccine is observed to inhibit the virus used for developing the vaccine when challenged with the same virus, but does not exhibit immunoprotection at all when challenged with the infected cells or viruses taken from a different patient. This viral diversity also remains as a problem yet to be solved.

There is therefore a pressing need for an AIDS drug that overcomes the problems encountered in the prior arts, including side effects and resistant viruses of conventional AIDS therapeutics.

### Disclosure

### Technical Problem

Leading to the present invention, intensive and thorough research into AIDS therapy, conducted by the present inventors, resulted in the finding that novel thiourea or urea derivatives have excellent inhibitory activity against HIV, without side effects and the induction of resistant viruses.

### Technical Solution

The present invention provides a novel thiourea or urea derivative, a method for preparing the same, and a pharmaceutical composition for the prophylaxis or therapy of AIDS, comprising the same as an active ingredient.

### Advantageous Effects

Having excellent inhibitory activity against HIV, the thiourea or urea derivatives of the present invention are useful in the prophylaxis or therapy of AIDS.

### Best Mode

In accordance with an aspect thereof, the present invention provides a thiourea or urea derivative, represented by the following Chemical Formula 1, or a pharmaceutically acceptable salt thereof: 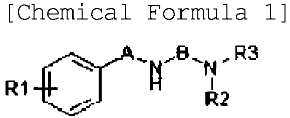 wherein,
A represents -CH₂ or -C(=O),
B represents -C(=O) or -C(=S),
R1 represents H, a halogen atom, linear or branched alkyl of C1~C4, phenyl, phenyl substituted with linear or branched alkyl of C1~C4, linear or branched alkoxy of C1~C4, aryloxy of C6~C20, or 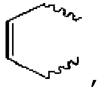 R2 and R3, which may be the same or different, independently represent H, linear or branched alkyl of C1~C4, cycloalkyl of C3~C8, -(linear or branched alkyl of C2~C6)-N-(alkyl of C1~C2)₂, -(linear or branched alkyl of C1~C4)-NH-(linear or branched alkyl of C1~C4), 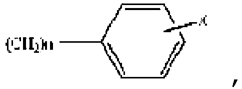 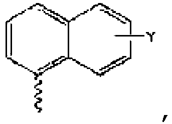 5,6,7,8-tetrahydronaphthalen-1-yl, (naphthalen-2-yl)-methyl, 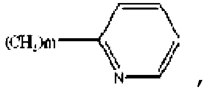 (pyridin-3-yl)-methyl, 2-chloro-pyridin-4-yl, pyrazin-2-yl, quinolin-5-yl, quinolin-8-yl, or 4-hydroxy-biphenyl-3-yl, or R2 and R3 are fused to each other, forming 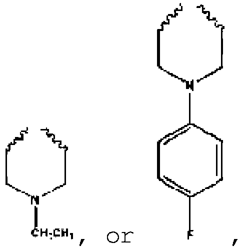 n is an integer of 0, or 1 to 4,
m is an integer of 0, 1 or 2,
X represents H, linear or branched alkyl of C1~C4, aryl of C6-C20, hydroxy, or a halogen atom, and
Y represents H or hydroxy,
with the provisos of the following conditions:
when A, B, and R1 represent -C(=O), -C(=S), and H,
respectively, to the exclusion of the options that R2 represents H, and R3 represents -CH₂-C(CH₃)₂-CH₂-N-(CH₃)₂, - (CH₂)₃-N-(CH₃)₂, phenyl, 4-methyl-benzyl, 4-chloro-benzyl, or (pyridin-2-yl)-ethyl, or R2 does not represent H, excluding the option that R2 and R3 are fused to each other, forming 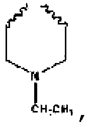 when A, B, and R1 represent -C(=O), -C(=S), and tert-butyl, respectively, to the exclusion of the option that R2 is H, and R3 represents phenyl, naphthalene, 3-chloro-phenyl, 3-toyl, 2-fluoro-phenyl or (pyridin-2-yl)-methyl,
when A, B, and R1 represent -C(=O), -C(=S), and methyl, respectively, to the exclusion of the option that R2 represents H, and R3 represents phenyl, naphthalene or pyridin-2-yl,
when A, B, and R1 represent -C(=O), -C(=S), and Cl, respectively, to the exclusion of the option that R2 represents H, and R3 represents cyclohexyl or naphthalene,
when A, B, and R1 represent -C(=O), -C(=O),and H, respectively, to the exclusion of the option that R2 represents H, and R3 represents benzyl.

In a preferred embodiment, the thiourea or urea derivative is represented by Chemical Formula 1 wherein,
R1 represents H, Cl, methyl, tert-butyl, phenyl, (tert-butyl)-phenyl, isopropoxy, phenoxy, or 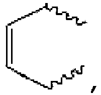 R2 and R3, which may be the same or different, independently represent H, methyl, cyclohexyl, -CH₂CH₂-N-(CH₃)₂, -CH₂CH₂CH₂-N- (CH₃)₂, -CH₂-C(CH₃)₂-CH₂-N-(CH₃)₂, -CH₂CH₂-N-(CH₂CH₃)₂, -CH (CH₃)CH₂CH₂CH₂-N-(CH₂CH₃)₂, - (CH₂)₃-NH- (CH₂CH₂CH₃), 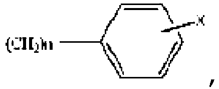 naphthalen-1-yl, 5-hydroxy-naphthalen-1-yl, 7-hydroxy-naphthalen-1-yl, 5,6,7,8-tetrahydronaphthalen-1-yl, pyridin-2-yl, (pyridin-2-yl)-methyl, (pyridin-2-yl)-ethyl, (pyridin-3-yl)-methyl, 2-chloro-pyridin-4-yl, pyrazin-2-yl, quinolin-5-yl, quinolin-8-yl, or 4-hydroxy-biphenyl-3-yl, or R2 and R3 are fused to each other, forming 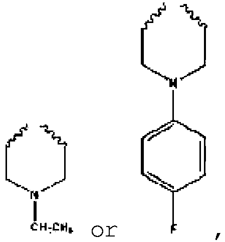 n is an integer of 0, or 1 to 4, and
X represents H, methyl, tert-butyl, phenyl, hydroxy, F, Cl, or Br.

Concrete examples of the compound represented by Chemical Formula 1 in accordance with the present invention include:
1) 1-(4-tert-butyl-benzoyl)-3-(5-hydroxy-naphthalen-1-yl)-thiourea,
2) 1-(4-tert-butyl-benzoyl)-3-(3-dimethylamino-propyl)-thiourea,
3) 1-(4-tert-butyl-benzoyl)-3-(3-dimethylamino-2,2-dimethyl-propyl)-thiourea,
4) 4-tert-butyl-N-(4-ethyl-piperazin-1-carbothioyl)-benzoamide,
5) 3-(4-tert-butyl-benzoyl)-1-(2-diethylamino-ethyl)-1-methyl-thiourea,
6) 3-(4-tert-butyl-benzoyl)-1-(2-dimethylamino-ethyl)-1-methyl-thiourea,
7) 1-(4-tert-butyl-benzoyl)-3-(4-diethylamino-1-methylbutyl)-thiourea,
8) 1-(4-tert-butyl-benzoyl)-3-(4-methyl-benzyl)-thiourea,
9) 1-(4-tert-butyl-benzoyl)-3-(2-methyl-benzyl)-thiourea,
10) 1-(4-tert-butyl-benzoyl)-3-(3-propylamino-propyl)-thiourea,
11) 1-(4-tert-butyl-benzoyl)-3-phenethyl-thiourea,
12) 1-(4-tert-butyl-benzoyl)-3-(4-chloro-benzyl)-thiourea,
13) 1-(4-tert-butyl-benzoyl)-3-(3-chloro-benzyl)-thiourea,
14) 1-(4-tert-butyl-benzoyl)-3-(naphthalen-2-ylmethyl)-thiourea,
15) 4-tert-butyl-N-[4-(4-fluoro-phenyl)-piperazin-1-carbothioyl]-benzamide,
16) 1-(4-tert-butyl-benzoyl)-3-cyclohexyl-thiourea,
17) 1-(4-tert-butyl-benzoyl)-3-(2-fluoro-benzyl)-thiourea,
18) 1-benzoyl-3-(naphthalen-2-ylmethyl)-thiourea,
19) 1-benzoyl-3-[2-(4-chloro-phenyl)-ethyl]-thiourea,
20) 1-benzoyl-3-(4-diethylamino-1-methyl-butyl)-thiourea,
21) N-[4-(4-fluoro-phenyl)-piperazin-1-carbothioyl]-benzamide,
22) 3-benzoyl-1-(2-diethylamino-ethyl)-1-methyl-thiourea,
23) N-(4-ethyl-piperazin-1-carbothioyl)-4-methyl-benzamide,
24) 1-cyclohexyl-3-(4-methyl-benzoyl)-thiourea,
25) 1-(4-chloro-benzyl)-3-(4-methyl-benzoyl)-thiourea,
26) 1-(3-dimethylamino-propyl)-3-(4-methyl-benzoyl)-thiourea,
27) 1-(4-methyl-benzoyl)-3-(3-methyl-benzyl)-thiourea,
28) 1-(4-methyl-benzoyl)-3-(4-phenyl-butyl)-thiourea,
29) 1-(4-methyl-benzoyl)-3-(3-phenyl-propyl)-thiourea,
30) 1-(3-dimethylamino-2,2-dimethyl-propyl)-3-(4-methylbenzoyl)-thiourea,
31) 1-benzoyl-3-[2-(4-fluoro-phenyl)-ethyl]-thiourea,
32) 1-(4-methyl-benzoyl)-3-(pyridin-3-ylmethyl)-thiourea,
33) 1-(4-methyl-benzoyl)-3-(pyridin-2-ylmethyl)-thiourea,
34) 1-[2-(3-bromo-phenyl)-ethyl]-3-(4-methyl-benzoyl)-thiourea,
35) 1-(4-methyl-benzoyl)-3-(2-pyridin-2-yl-ethyl)-thiourea,
36) 1-(4-tert-butyl-benzoyl)-3-(naphthalen-1-yl)-thiourea,
37) 1-(4-tert-butyl-benzoyl)-3-(pyridin-2-yl)-thiourea,
38) 1-(4-tert-butyl-benzoyl)-3-(3-chloro-phenyl)-thiourea,
39) 1-(4-methyl-benzoyl)-3-(naphthalen-1-yl)-thiourea,
40) 1-(4-chloro-benzoyl)-3-(3-dimethylamino-2,2-dimethylpropyl)-thiourea,
41) 1-(4-chloro-benzoyl)-3-(naphthalen-2-ylmethyl)-thiourea,
42) 1-(4-chloro-benzoyl)-3-(5-hydroxy-naphthalen-1-yl)-thiourea,
43) 1-(4-phenoxy-benzoyl)-3-(pyridin-2-yl)-thiourea,
44) 1-(biphenyl-4-carbonyl)-3-(3-dimethylamino-2,2-dimethyl-propyl)-thiourea,
45) 1-(4'-tert-butyl-biphenyl-4-carbonyl)-3-(3-chloro-phenyl)-thiourea,
46) 1-(3-dimethylamino-2,2-dimethyl-propyl)-3-(naphthalene-2-carbonyl)-thiourea,
47) 1-(biphenyl-4-carbonyl)-3-(3-chloro-phenyl)-thiourea,
48) 1-(4-tert-butyl-benzoyl)-3-(naphthalen-1-yl)-thiourea,
49) 1-(4-tert-butyl-benzoyl)-3-(5,6,7,8-tetrahydro-naphthalen-1-yl)-thiourea,
50) 1-(4-tert-butyl-benzoyl)-3-(quinolin-5-yl)-thiourea,
51) 1-(4-tert-butyl-benzoyl)-3-(4-tert-butyl-phenyl)-thiourea,
52) 1-(3-dimethylamino-2,2-dimethyl-propyl)-3-(4-isopropoxy-benzoyl)-thiourea,
53) 1-(4-isopropoxy-benzoyl)-3-(naphthalen-1-yl)-thiourea,
54) 1-(5-hydroxy-naphthalen-1-yl)-3-(4-isopropoxy-benzoyl)-thiourea,
55) 1-(4-isopropoxy-benzoyl)-3-(pyridin-2-yl)-thiourea,
56) 1-(4-isopropoxy-benzoyl)-3-(m-tolyl)-thiourea,
57) 1-(4-tert-butyl-benzoyl)-3-(4-phenyl-butyl)-thiourea,
58) 1-(4-tert-butyl-benzoyl)-3-(4-phenyl-propyl)-thiourea,
59) 1-(4-tert-butyl-benzoyl)-3-[2-(2-chloro-phenyl)-ethyl)-thiourea,
60) 1-(4-tert-butyl-benzoyl)-3-(3-methyl-benzyl)-thiourea,
61) 1-(4-tert-butyl-benzoyl)-3-(quinolin-8-yl)-thiourea,
62) 1-(4-tert-butyl-benzoyl)-3-(m-tolyl)-thiourea,
63) 1-(4-tert-butyl-benzoyl)-3-(pyrazin-2-yl)-thiourea,
64) 1-(4-tert-butyl-benzoyl)-3-(3-hydroxy-phenyl)-thiourea,
65) 1-(4-tert-butyl-benzoyl)-3-(7-hydroxy-naphthalen-1-yl)-thiourea,
66) 1-(4-tert-butyl-benzoyl)-3-(2-chloro-pyridin-4-yl)-thiourea,
67) 1-(4-tert-butyl-benzoyl)-3-(4-hydroxy-biphenyl-3-yl)-thiourea,
68) 1-biphenyl-3-yl-3-(4-tert-butyl-benzoyl)-thiourea,
69) 1-(4-tert-butyl-benzoyl)-3-(2-fluoro-phenyl)-thiourea,
70) N-(2-fluorophenylcarbamothioyl)-4-isopropoxybenzamide,
71) N-(4-tert-butylphenylcarbamothioyl)-4-isopropoxybenzamide,
72) N-(biphenyl-3-ylcarbamothioyl)-4-isopropoxybenzamide,
73) N-(7-hydroxynaphthalen-1-ylcarbamothioyl)-4-isopropoxybenzamide,
74) 1-(4-tert-butyl-benzyl)-3-(5-hydroxy-naphthalen-1-yl)-thiourea,
75) 1-(4-tert-butyl-benzyl)-3-(5-hydroxy-naphthalen-1-yl)-urea,
76) 1-(4-tert-butyl-benzyl)-3-(3-chloro-phenyl)-urea,
77) 1-(4-tert-butyl-benzyl)-3-cyclohexyl-urea,
78) 1-(4-tert-butyl-benzoyl)-3-(5-hydroxy-naphthalen-1-yl)-urea,
79) 1-(4-tert-butyl-benzoyl)-3-(pyridin-2-yl)-urea,
80) 1-(3-dimethylamino-2,2-dimethyl-propyl)-3-(4-methylbenzoyl)-urea,
81) 1-cyclohexyl-3-(4-methyl-benzoyl)-urea,
82) 1-(4-methyl-benzoyl)-3-(naphthalen-2-ylmethyl)-urea,
83) 1-benzoyl-3-(3-dimethylamino-2,2-dimethyl-propyl)-urea, and
84) 3-benzoyl-1-(2-dimethylamino-ethyl)-1-methyl-urea.

The thiourea or urea derivatives of Chemical Formula 1 according to the present invention may be prepared into pharmaceutically acceptable salts or solvates using typical methods known in the art.

Within the range of the pharmaceutically acceptable salts are included, for example, acid addition salts formed with pharmaceutically acceptable free acids. The acid addition salts of the compound according to the present invention may be prepared using a conventional method, for example, by dissolving the thiourea or urea derivative in excess acid in water and precipitating the resulting salt in a water-miscible organic solvent, e.g., methanol, ethanol, acetone or acetonitrile. Alternatively, the thiourea or urea derivative of the present invention may be heated along with the same molar amount of acid or alcohol (e.g., glycol monomethyl ether) in water, followed by evaporating the mixture and drying or suction filtering the precipitate to prepare acid addition salts thereof.

The thiourea or urea derivatives of the present invention may be prepared into pharmaceutically acceptable salts or solvates using typical methods known in the art.

Within the range of the pharmaceutically acceptable salts are included, for example, acid addition salts formed with pharmaceutically acceptable free acids. The acid addition salts of the compound according to the present invention may be prepared using a conventional method, for example, by dissolving the thiourea or urea derivative in excess acid in water and precipitating the resulting salt in a water-miscible organic solvent, e.g., methanol, ethanol, acetone or acetonitrile. Alternatively, the thiourea or urea derivative of the present invention may be heated along with the same molar amount of acid or alcohol (e.g., glycol monomethyl ether) in water, followed by evaporating the mixture and drying or suction filtering the precipitate to prepare acid addition salts thereof.

The free acids may be inorganic or organic. Examples of useful inorganic free acids include hydrochloric acid, phosphoric acid, sulfuric acid, nitric acid, and stannic acid. As organic acids, methane sulfonic acid, p-toluene sulfonic acid, acetic acid, trifluoroacetic acid, citric acid, maleic acid, succinic acid, oxalic acid, benzoic acid, tartaric acid, fumaric acid, mandelic acid, propionic acid, lactic acid, glycollic acid, gluconic acid, galacturonic acid, glutamic acid, glutaric acid, glucuronic acid, aspartic acid, ascorbic acid, carbonic acid, vanillic acid, hydroiodic acid may be used.

Also, metal salts formed with bases may fall within the range of pharmaceutically acceptable salts of the compound of the present invention. Examples of the metal salts useful in the present invention include alkali metal salts and alkaline earth metal salts. By way of example, the compound of the present invention may be dissolved in excess alkali metal hydroxide or alkaline earth metal hydroxide in water, and, after the filtration of the solution to remove non-dissolved compound salts, the filtrate may be dried to afford the pharmaceutically acceptable salts of the compound of the present invention. Suitable for use in pharmaceutics are sodium, potassium or calcium salts. Corresponding silver salts may be obtained by reacting the alkali metal or alkaline earth metal salts with suitable silver salt (e.g., silver nitrate).

Unless otherwise stated, the pharmaceutically acceptable salts of the thiourea or urea derivatives of the present invention include salts of the acid or base groups present on the thiourea or urea derivatives. For example, the pharmaceutically acceptable salts include sodium, calcium or potassium salts of hydroxyl groups. For amino groups, hydrobromide, sulfate, hydrogen sulfate, phosphate, hydrogen phosphate, dihydrogen phosphate, acetate, succinate, citrate, tartrate, lactate, mandelate, methanesulfonate (mesylate), and *p*-toluenesulfonate (tosylate) may be exemplified, and can be prepared using a typical method known in the art.

In accordance with another aspect thereof, the present invention addresses a method for preparing the thiourea or urea derivative of Chemical Formula 1, as illustrated in one of Reaction Schemes 1 to 5.

In one embodiment, when A is -C(=O),and B is -C(=S) in Chemical Formula 1, the method for preparing the thiourea or urea derivative of Chemical Formula 1 comprises:
1) reacting a carboxylic acid compound of Chemical Formula 2 with oxalyl chloride in an organic solvent to synthesize a compound of Chemical Formula 3,
2) reacting the compound of Chemical Formula 3, synthesized in 1), with potassium thiocyanate (KSCN) in an organic solvent to synthesize a compound of Chemical Formula 4, and
3) reacting the compound of Chemical Formula 4, synthesized in 2), with an amine compound of Chemical Formula 4 to afford a compound of Chemical Formula 1-1, as illustrated in the following Reaction Scheme 1: 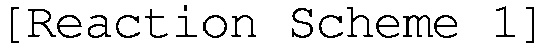 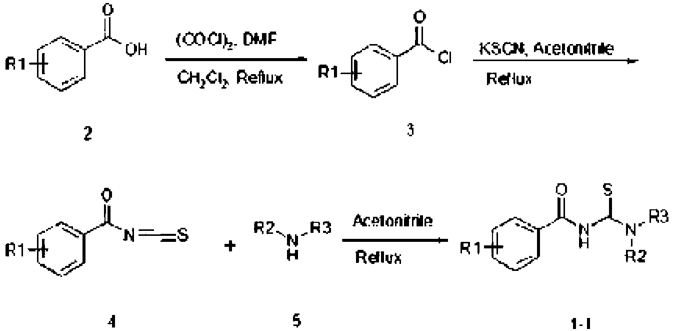
wherein R1, R2, and R3 are as defined in Chemical Formula 1.

In another embodiment, when A is -CH₂, and B is -C(=S) in Chemical Formula 1, the method for preparing the thiourea or urea derivative of Chemical Formula 1 comprises mixing an amine compound of Chemical Formula 5 with sodium hydrogen carbonate in an organic solvent, and reacting the mixture with thiophosgen and an amine compound of Chemical Formula 6 to afford a compound of Chemical Formula 1-2, as illustrated in the following Reaction Scheme 2: 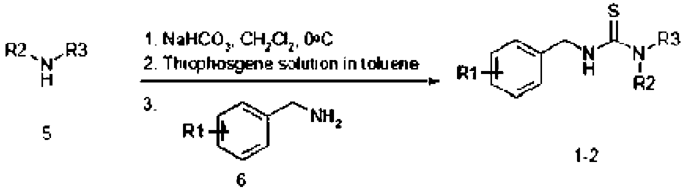 wherein R1, R2 and R3 are as defined in Chemical Formula 1, above.

In a further embodiment, when A is -CH₂, and B is -C(=O) in Chemical Formula 1, the method for preparing the thiourea or urea derivative of Chemical Formula 1 comprises mixing an amine compound of Chemical Formula 5 with sodium hydrogen carbonate in an organic solvent and reacting the mixture with phosgene and an amine compound of Chemical Formula 6 to afford a compound of Chemical Formula 1-3, as illustrated in Reaction Scheme 3: 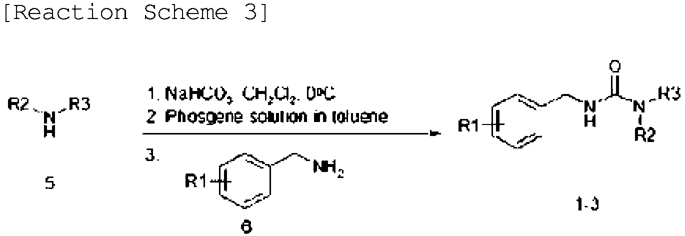 wherein, R1, R2, and R3 are as defined in Chemical Formula 1, above.

In a still further embodiment, when A is -C(=O) and B is -C(=O) in Chemical Formula 1, the method for preparing the thiourea or urea derivative of Chemical Formula 1 comprises
1) reacting a carboxylic acid compound of Chemical Formula 2 with 1,1'-carbonyldiimidazole in an organic solvent to synthesize a compound of Chemical Formula 7, and
2) mixing an amine compound of Chemical Formula 5 with sodium hydrogen carbonate in an organic solvent, and then reacting the mixture with phosgene and the compound of Chemical Formula 7, synthesized in 1), to afford a compound of Chemical Formula 1-4, as illustrated in the following Reaction Scheme 4: 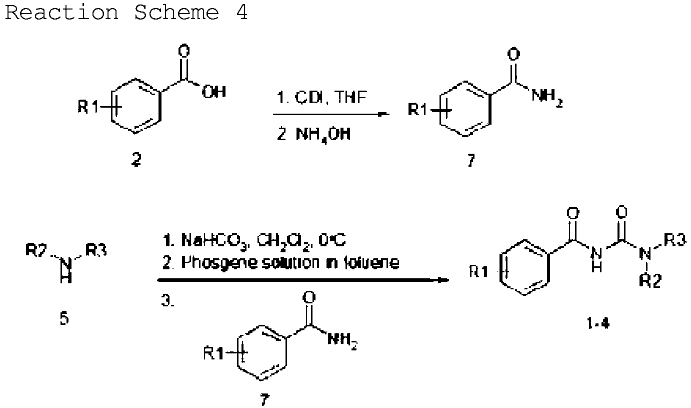
wherein, R1, R2, and R3 are as defined in Chemical Formula 1, above.

In a still yet further embodiment, when A is -C(=O),and B is -C(=O) in Chemical Formula 1, the method for preparing the thiourea or urea derivative of Chemical Formula 1 comprises:
1) reacting a carboxylic acid compound of Chemical Formula 2 with oxyalyl chloride in an organic solvent to synthesize a compound of Chemical Formula 3, and
2) reacting the compound of Chemical Formula 3, synthesized in 1), with tin chloride (SnCl4), sodium cyanate (NaOCN), and an amine compound of Chemical Formula 5 to afford a compound of Chemical Formula 1-4, as illustrated in the following Reaction Scheme 5: 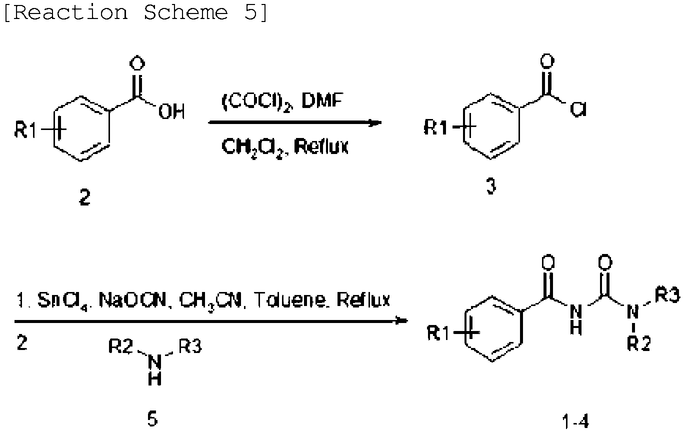
wherein, R1, R2, and R3 are as defined in Chemical Formula 1, above.

The organic solvent used in Reaction Schemes 1 to 5 may be selected from the group consisting of, but not limited to, methylene chloride, acetonitrile, tetrahydrofuran, toluene, and a combination thereof.

In accordance with a further aspect thereof, the present invention provides a pharmaceutical composition for the prevention or treatment of AIDS, comprising the thiourea or urea derivative of Chemical Formula 1 as an active ingredient.

In a preferred embodiment, the thiourea or urea derivative is selected from the group consisting of:
85) 4-tert-butyl-N-(4-methyl-piperazin-1-carbothioyl)-benzamide,
86) 1-benzoyl-3-(3-dimethylamino-2,2-dimethyl-propyl)-thiourea,
87) 1-benzoyl-3-(4-methyl-benzyl)-thiourea,
88) 1-benzoyl-3-(3-dimethylamino-propyl)-thiourea,
89) N-(4-ethyl-piperazin-1-carbothioyl)-benzamide,
90) 1-benzoyl-3-(4-chloro-benzyl)-thiourea,
91) 1-benzoyl-3-phenyl-thiourea,
92) 1-benzoyl-3-(2-pyridin-2-yl-ethyl)-thiourea,
93) 1-(4-tert-butyl-benzoyl)-3-phenyl-thiourea,
94) 1-(4-tert-butyl-benzoyl)-3-(pyridin-2-ylmethyl)-thiourea,
95) 1-(4-tert-butyl-benzoyl)-3-(pyridin-4-ylmethyl)-thiourea,
96) 1-(4-methyl-benzoyl)-3-(pyridin-2-yl)-thiourea,
97) 1-(4-methyl-benzoyl)-3-phenyl-thiourea,
98) 1-(4-chloro-benzoyl)-3-thiohexyl-thiourea,
99) 1-(4-chloro-benzoyl)-3-(naphthalen-2-ylmethyl)-thiourea, and
100) 1-benzoyl-3-benzyl-urea.

The thiourea or urea derivatives of the present invention exhibit excellent anti-HIV activity by inhibiting HIV viral expression. Hence, the thiourea or urea derivatives of the present invention may be useful for preventing or treating AIDS.

The composition of the present invention may further comprise at least one active ingredient known for inhibitory activity against HIV, in addition to the thiourea or urea derivative.

For administration, the pharmaceutical composition may further comprise a pharmaceutically acceptable carrier, in addition to the active ingredients. Examples of the pharmaceutically acceptable carrier include saline, sterile water, Ringer's solution, buffered saline, a dextrose solution, a maltodextrin solution, glycerol, ethanol, etc. Optionally, typical additives, such as antioxidants, buffers, bacteriostatic agents, etc., may be added to the composition. For the preparation of dosage forms including injections, such as aqueous solutions, suspensions and emulsions, pills, capsules, granules and tablets, the active ingredients may be admixed with a diluent, a dispersant, a surfactant, a binder and/or a lubricant. Reference may be made to literature (Remington's Pharmaceutical Science (latest edition), Mack Publishing Company, Easton PA) regarding the formulation of the pharmaceutical composition into suitable dosage forms.

The composition of the present invention may be administered via oral routes or parenteral routes (e.g., intravenous, subcutaneous, intraperitoneal, topical, etc.). The effective dosage of the inhibitor in accordance with the present invention depends on various factors, including the patient's weight, age, gender, state of health, diet, the time of administration, the route of administration, excretion rate, severity of diseases, etc. In general, the thiourea or urea derivative may be administered in a single dose, and preferably in multiple doses per day at a daily dose ranging from 0.1 to 50 mg/day, and preferably from 2 to 10 mg/kg.

For the effective prophylaxis and therapy of AIDS, the composition according to the present invention may be used alone or in combination with surgical operation, hormonal therapy, a drug, and/or biological response controllers.

A better understanding of the present invention may be obtained through the following examples which are set forth to illustrate, but are not to be construed as limiting the present invention.

### EXAMPLE 1: Preparation of 1-(4-tert-Butyl-benzoyl)-3-(5-hydroxy-naphthalen-1-yl)-thiourea According to Reaction Scheme 1

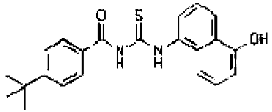

### 1. Synthesis of 4-(tert-butyl)-benzoyl chloride

At room temperature, 4-(tert-butyl)-benzoic acid (0.5 g, 2.80 mmol) was mixed with methylene chloride (28 mL, 0.1 M) and 2M oxalyl chloride (4.2 mL, 8.4 mmol), and two or three drops of dimethyl formamide were added to the mixture, followed by stirring at 70°C for 6 hrs. Concentration in vacuo yielded the title compound.

### 2. Synthesis of 4-(tert-butyl)-benzoyl isothiocyanate

At room temperature, 4-(tert-butyl)-benzoyl chloride, synthesized in 1, was mixed with acetonitrile (24 mL, 0.2 M) and potassium thiocyanate (1.08 g, 11.2 mmol) at 70°C for 12 hrs with stirring. Then, the reaction mixture was cooled to room temperature, and after removal of salt by filtration, concentration in vacuo afforded the title compound.

### 3. Synthesis of 1-(4-tert-butyl-benzoyl)-3-(5-hydroxy-naphthalen-1-yl)-thiourea

At room temperature, 4-(tert-butyl)-benzoyl isocyanate, synthesized in 2, was mixed with acetonitrile (24 mL, 0.2 M) and 5-amino-naphthalen-1-ol (0.89 g, 5.60 mmol) under flux. Methylene chloride was added to this mixture, and the salt thus formed was removed, followed by concentration under a reduced pressure. The concentrate was purified by silica gel column chromatography to afford the title compound (0.37 g, Yield: 36.43 %).
¹H NMR (500MHz, CDCl₃): 12.78(s, 1H), 9.27(s, 1H), 8.23(d, J=8.5Hz, 1H), 8.08(d, J=8.0Hz, 1H), 7.91(d, J=8.5Hz, 2H), 7.63-7.55(m, J=4H), 7.40(t, J=8.5Hz, 1H), 6.88(d, J=7.0Hz, 1H), 1.39(s, 9H)
LC-MS (M⁺H): 379

### EXAMPLES 2 TO 73

The same procedure as in Example 1 was carried out to prepare compounds of Examples 2 to 73. In Table 1, structural formulas, MS data, and ¹H NMR data of the compounds of Examples 2 to 73 are summarized.

**TABLE 1**

| 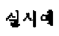 | 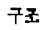 | LC-MS (M⁺H) | ¹H NMR |
|---|---|---|---|
| 2 | 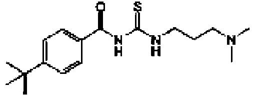 | 322 | ¹H NMR(500MHz, CDCl₃) 11.00(s, 1H). 8.96(s, 1H). 7.79-7.76(m, 2H). 7.54-7.52(m. 2H), 3.84(t, J=6.7Hz, 2H), 2,70(t, J=7.0Hz, 2H), 2.49(s, 6H), 2.07(t, J=7.5Hz. 2H), 1.36(s, 9H) |
| 3 | 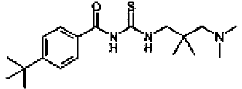 | 350 | ¹H NMR(500MHz. CDCl₃) 8.93(s. 1H). 7.78(d, J=8.0Hz, 2N). 7.51(d, J=8.0Hz, 2H), 3.66(s, 2H), 2.37(s, 8H). 1.34(s, 9H). 1.05(s, 6H) |
| 4 | 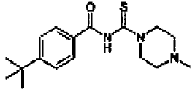 | 334 | |
| 5 | 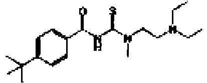 | 350 | ¹H NMR(500MHz, DMSO-d₆) 7.88-7.76(m, 2H), 7.53-7.44(m, 2H), 3.96(t. J=7.0Hz, 2H), 3.15(s, 3H), 2.59-2.57(m, 4H), 3.47-2.43(m, 2H), L.30(s, 9H), 1.01(t, J=7.0Hz, 3H), 0.88(t, J=7.0H₂, 3H) |
| 6 | 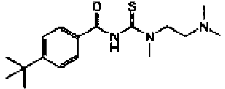 | 322 | ¹H NMR(500MHz, DMSO-d₆) 7.91-7.80(m, 2H), 7.53-7.41(m. 2H), 4.05(t, J=6.8Hz. 2H). 3.15(s, 3H), 2.77(s, 2H), 2.34(s, 3H). 2.15(s, 3H), 1.30(s, 9H) |
| 7 | 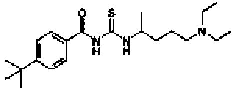 | 378 | ¹H NMR(500MHz, DMSO-d₆) 11.45(s, 1H), 10.85(t, J=8.0Hz, 1H), 7.89(d, J=8.5Hz, 2H), 7.53(d, J=8.5Hz, 2H), 4.41(t, J=6.5Hz. 1H), 2.62(s, 6H), 1.64-1.56(m, 2H), 1.52-1.49(m, 2H), 1.30(s, 9H), 1.23(d, J=6.5Hz, 3H), 1.00(t, J=7.0Hz. 6H) |
| 8 | 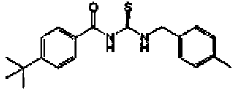 | 341 | ¹H NMR(500MHz, CDCl₃) 11.01(s, 1H), 9.01(s, 1H), 7.77(d, J=8.5Hz. 2H), 7.53(d, J=8.5Hz, 2H), 7.30(d, J=8.0Hz, 2H), 7.20(d, J=8.0Hz, 2H), 4.88(d, J=5.0Hz. 2H), 2.37(s, 3H), 1.36(s, 9H) |
| 9 | 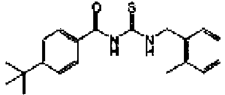 | 34] | ¹H NMR(500MHz, CDCl₃) 10.93(s, 1H), 9.06(s, 1H), 7.78(d, J=10.5Hz, 2H), 7.53(d, J=10.5Hz, 2H), 7.35(d, J=7.5Hz, 1H), 7.28-7.21(m, 3H), 4.90( d, J=5.0Hz, 2M), 2.41(s, 3H), 1.36(s, 9H) |
| 10 | 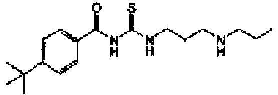 | 336 | ¹H NMR(500MHz, CDCl₃) 7.79(d, J=8.5Hz, 2H), 7.55(d, J=8.5Hz, 2H), 3,97(t, J=6.0Hz, 2H), 3.02(t. J=6.2Hz, 2H), 2-90(t, J=7.8Hz, 2H), 2.26(t. J=6.0Hz, 2H), 1.84-1.83(m. 2H), 1.36(s, 9H), 1.06(t, J=7.5Hz, 3H) |
| 11 | 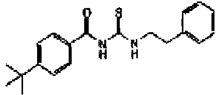 | 341 | ¹H NMR(500MHz, CDCl₃) 10.82(s, 1H), 8.97(s, 1H). 7.77(d, J=11.0Hz, 2H), 7.53(d, J=9.0Hz, 2H), 7.37-7.34(m, 2H. 7.31-7.25(m, 3H), 4.00-3.96(m. 2H), 3.05(t, J=7.5Hz, 2H), 1.36(s, 9H) |
| 12 | 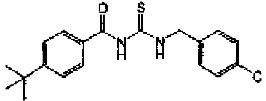 | 361 | ¹H NMR(500MHz, CDCl₃) 11.09(s, 1H), 9.06(s, 1H), 7.78(d, J=6,5Hz, 2H), 7.53(d, J=8.5Hz, 2H). 7.35(s. 4H). 4.90(d, J=5.5Hz, 2H). 1.36(s, 9H) |
| 13 | 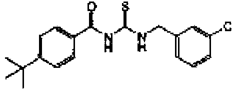 | 361 | ¹H NMR(500MHz, CDCl₃) 11.12(s, 1H), 9.12(s, 1H), 7.78(d, J=8.5Hz 2H), 7.52(d, J=8.5Hz, 2H), 7.39(s, 1H), 7.33-7.27(m, 3H), 4.92(d, J=5.5Hz, 2H), 1.35(s, 9H) |
| 14 | 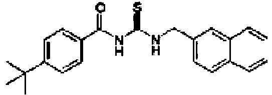 | 377 | |
| 15 | 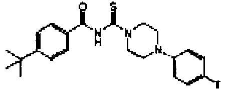 | 400 | ¹H NMR(500MHz, CDCl₃) 8.35(s. 1H). 7,72(d. J=10.5Hz, 2H), 7,44(d. J=8.5Hz, 2H), 6.94-6.90(m 2H), 6,84-6.92(m. 2H), 4.31(s, 2H), 3,74(s, 2H), 3.20(d, J=25,5Hz, 4H), 1.28(s, 9H) |
| 16 | 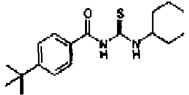 | 319 | |
| 17 | 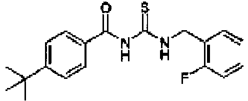 | 345 | ¹H NMR(500MHz, CDCl₃) 7.78(d. J=8.0Hz, 2H), 7.52(d, J=8.5Hz, 2H), 7.47(t, J=7,5Hz, 1H), 7,34-7.28(m, 1H), 7.16(t, J=7.5Hz, 1H), 7,11(t. J=9.5Hz, 1H), 4.99(d, J=5.5Hz, 2H), 1.36(s, 9H) |
| 18 | 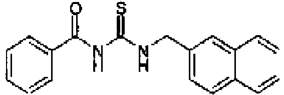 | 321 | ¹H NMR(500MHz, CDCl₃) 10.92(s. 1H), 8.98(s, 1H), 7.99(d, J=8.5Hz, 1H), 7.84(d, J=8.0Hz 1H). 7.79(d, J=8.0Hz, 1H). 7.72(d, J=7.5Hz, 2H), 7.55-7.3(m, 7H). 5.28(d. J=5-5Hz, 2H) |
| 19 | 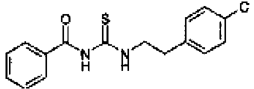 | 319 | ¹H NMR(500MHz, CDCl₃) 10.69(s, 1H), 8.88(s, 1H), 7,75(d, J=8.0Hz. 2H), 7,57(t, J=7.0Hz, 1H), 7.45(t, J=7.5Hz. 2H). 7.24(d, J=8.5Hz. 2H), 7.15(d, J=8.0Hz, 2H), 3.90-3.86(m, 2H), 2.96(t, J=7,0Hz. 2H) |
| 20 | 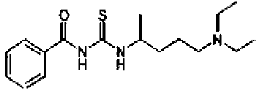 | 322 | ¹H NMR(500MHz, CDCl₃) 10.61(d, J=8.0Hz, 1H), 7.78(d, J=7.5Hz, 2H), 7.57(t, J=7.5Hz. 1H), 7.46(t, J=7.7Hz, 2H), 4.52-4.48(m, 1H), 3,10-2.90(m, 6H), 1.88-1.79(m, 4H), 1.31-1.27(m. 9H) |
| 21 | 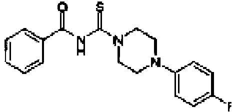 | 344 | ¹H NMR(500MHz, CDCl₃) 8.36(s, 1H), 7.79(d, J=7.5Hz. 2H), 7.55(t, J=7.5Hz. 1H), 7.45(t, J=7.8Hz, 2H), 6.95-6,90(m, 2H), 6.86-6.83(m, 2H), 4.32(s, 2H), 3.76(s, 2H), 3.22(d, J=25.0Hz, 4H) |
| 22 | 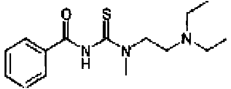 | 294 | |
| 23 | 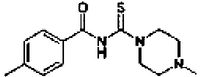 | 292 | ¹H NMR(500MHz, CDCl₃) 8.43(s, 1H), 7.67-7.65(m, 2H), 7.20(d, J=7.5Hz, 2H), 4.18(s, 2H), 3.60(s, 2H), 2.53(d, J=30,5Hz, 1H), 2.43-2.38(m, 2H), 2.35(s, 3H), 1.02(t, J=7.3Hz, 3H) |
| 24 | 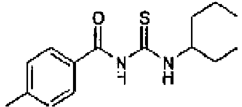 | 277 | ¹H NMR(500MH, CDCl₃) 10.68(s, 1H), 8.80(s. 1H), 7.65(d, J=8.5Hz, 2H), 7.24(d, J=8.5Hz, 2H), 424-4.21(m. 1H). 2.36(s, 3H), 2.02(d, J=9.5Hz, 2H), 1.69-1.63(m, 2H), 1.57-1.54(m, 1H), 1.42-1.30(m. 4H), 1.23-1.21(m, 1H) |
| 25 | 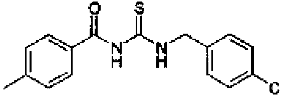 | 319 | ¹M NMR(500MHz, CDCl₃) 11.00(s. 1H), 8.95(s, 1H). 7.66(d, J=8.5Hz, 2H), 7.27(s, 3H), 7.26-7,232(m, 3H), 4.83(d. J=5.5Hz, 2H), 2.37(s, 3H) |
| 26 | 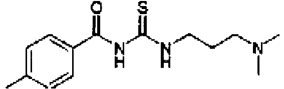 | 280 | ¹H NMR(500MHz, CDCl₃) 10.96(s, 1H), 8.88(s, 1H), 7.66(d, J=9.5Hz, 2H), 7.23(d, J=8.0Hz. 2H), 3.74-3.70(m, 2H), 2.36-2.22(m, 5H), 2.19(s, 6H), 1.79(t, J=6.7Hz. 2H) |
| 27 | 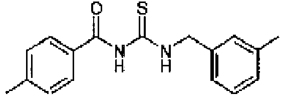 | 299 | ¹H NMR(500MHz, CDCl₃) 10.96(s, 1H), 8.98(s. 1H), 7.67(d. J=8.0Hz, 2H), 7.24(d, J=8.5Hz, 2H), 7.20(1, J=7.5Hz, 1H), 7.13(d- J=8,0Hz, 2H), 7.07(d, J=7,5Hz. 1H), 4,82(d, J=5.5Hz, 2H), 2.37(s, 3H), 2.31(s, 3H) |
| 28 | 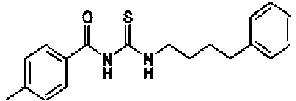 | 327 | ¹H NMR(500MHz, CDCl₃) 10.72(s, 1H), 8.90(s, 1H), 7.66(d. J=8.0Hz. 2H), 7.26-7.20(m, 3H), 7.14(d, J=7.0Hz, 2H), 3.69-3.66(m, 2H), 2.64-2.62(m, 2H), 2.38(s, 3H), 1.71-1.68 (m. 4H) |
| 29 | 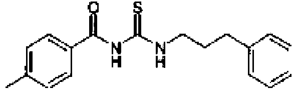 | 313 | ¹H NMR(500MHz, CDCl₃) 10.75(s. 1H). 8.89(s, 1H), 7.66(d. J=8.0Hz, 2H), 7.25-7.22(m. 4H). 7.16-7.12(m. 3H), 3.69-3.65(m. 2H), 3.68(t, J=7.5Hz. 2H), 2.37(s, 3H), 2.03-1.97(m, 2H) |
| 30 | 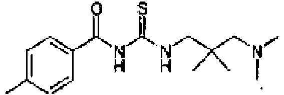 | 308 | ¹H NMR(500MHz. CDCl₃) 11.55(s. 1H), 8.85(s, 1H), 7.67(d, J=8.0Hz, 2H), 7.22(d. J=8.0Hz. 2H), 3.57(d. J=4.5Hz, 2H), 2.36(s, 3H). 2.26(s, 6H), 2.22(s, 2H), 0.94(s, 6H) |
| 31 | 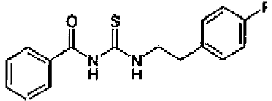 | 303 | ¹H NMR(500MHz, CDCl₃) 10.77(s, 1H), 9.03(s, 1H), 7.83-7.81(m, 2H), 77.65-7.61(m, 1H), 7.53-7.50(m. 2H), 7.28-7.24(m. 2H), 7.06-7.02(m, 2H). 3.97-3.93(m. 2H), 3.02(t, J=7,2Hz. 2H) |
| 32 | 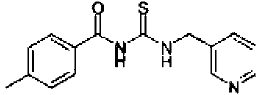 | 286 | ¹H NMR(500MHz, CDCl₃) 11-56(s. 1H), 9.26(s, 1H), 8.64(s, 1H). 8.56-8,55(m, 1H), 7,76-7.71(m, 3H), 7.30-7.28(m. 3H), 4,94(d. J=6.0Hz, 2H), 2.42(s, 3H) |
| 33 | 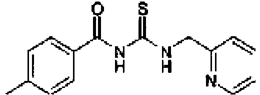 | 286 | ¹H NMR(500MHz, CDCl₃) 11.68(s.1H). 9.07(s, 1H). 8.68-8.67(m, 1H), 7,79(d, J=8.5Hz, 2H), 7.41(d, J=8.0Hz, 1H), 7.33(d, J=8.5Hz, 2J). 7.30(d, J=5.5Hz, 1H), 5.31(s, 1H), 5.08(d, J=4.5Hz, 2H), 2.45(s, 3H) |
| 34 | 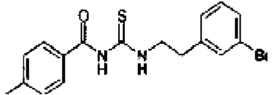 | 377 | ¹H NMR(500MHz. CDCl₃) 10.82(s, 1H), 8,96(s, 1H), 7.73(d, J-8.0Hz, 2H), 7.45(s, 1H), 7.41-7.39(m, 1H), 7.32(d, J-8.5Hz, 2H), 7.24-7.22(m, 2H) |
| 35 | 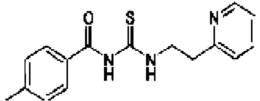 | 300 | ¹H NMR(500MHz. CDCl₃) 11.09(s, 1H), 8,94(s, 1H), 8.64(d, J=4.0Hz, 1H), 7.73(d, J=8.5Hz, 3H), 7.31(d, J=8.0Hz, 3H), 7.26-7.24(m, 1H), 4.21-4.17(m, 2H), 3.39(t, J=6.5Hz, 2H), 2.44(s, 3H) |
| 36 | 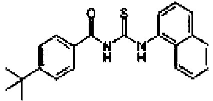 | 363 | ¹H NMR(500MHz, CDCl₃) 12.82(s, 1H), 9.28(s, 1H), 8.08(m, 2H), 7.91(d, 1=8.5Hz, 2H), 7.87(d, J=8.0Hz, 1H), 7.62-7.54(m, 6H), 1.40(s, 9H) |
| 37 | 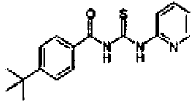 | 314 | |
| 38 | 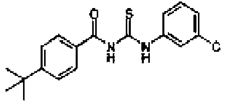 | 347 | ¹H NMR(500MHz, CDCl₃) 12.65(s, 1H), 9.01(s, 1H), 7.80(t, J-2.0Hz, 1H), 7.77-7.74(m, 2H), 7.54-7.52(m, 1H), 7.50-7.48(m, 2H), 7.28(t, J=8.0Hz, 2H), 7.20-7.18(m, 2H), 1.30(s, 9H) |
| 39 | 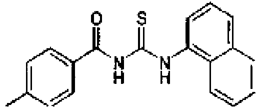 | 321 | ¹H NMR(500MHz, CDCl₃) 12.81(s, 1H), 9.27(s, 1H), 8.32-8.29(m, 1H), 8.05-8.01(m, 2H), 7.89-7.76(m, 3H), 7.61-7.53(m, 3H), 7.45-7.33(m, 2H), 2.49(s, 3H) |
| 40 | 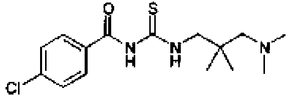 | 328 | |
| 41 | 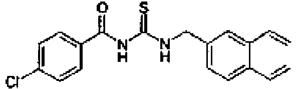 | 355 | ¹H NMR(50OMHz, CDCl₃) 10.91(s, 1H), 9.00(s, 1H), 8.05(d, J=8.0Hz, 1H), 7.87-7.71(m, 4H), 7.58-7.47(m, 6H), 5.36(d, J=5.0Hz, 2H) |
| 42 | 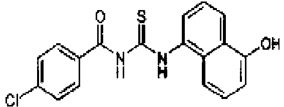 | 357 | |
| 43 | 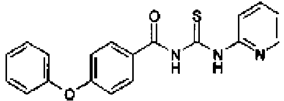 | 350 | ¹H NMR(500MHz, CDCl₃) 8.76-8.74(m, 1H), 8.18-8.05(m, 2H), 7.42(t, J-4.2Hz, 3H) |
| 44 | 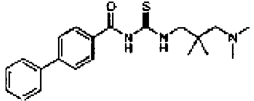 | 370 | ¹H NMR(500MHz, CDCl₃) 11.65(s, 1H), 9.00(s, 1H), 7.95-7.92(m, 2H), 7.74(d, J=9.0Hz, 2H), 7.65-7.64(m, 2H), 7.50(t, J=7.5Hz, 2H), 7.45-7.42(m, 1H), 3.69(s, 2H), 2.38(s, 8H), 1.06(s, 6H) |
| 45 | 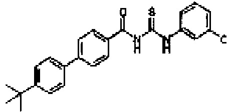 | 423 | ¹H NMR(500MHz, CDCl₃) 12.73(s, 1H), 9.14(s, 1H), 7.98-7.96(m, 2H), 7.90-7.89(m, 1H), 7.79-7.77(m, 2H), 7.63(s, 1H), 7.62-7.6(m, 2H), 7.55-7.53(m, 2H), 7.37(tn J-8.0Hz, 1H), 7.29(s, 1H), 1.40(s, 9H) |
| 46 | 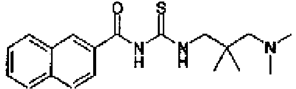 | 344 | ¹H NMR(500MHz, CDCl₃) 11.66(s, 1H), 9.18(s, 1H), 8.39(s, 1H), 7.96(t, J=7.8Hz, 2H), 7.92-7.87(m, 2H), 7.66-7.58(m, 2H), 3.70(d, J=5.0Hz, 2H), 2.42(s, 8H), 1.07(s, 6H) |
| 47 | 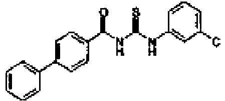 | 367 | ¹H NMR(500MHz, CDCl₃) 11.62(s, 1H), 9.04(s, 1H), 7.94-7.91(m, 2H), 7.74-7.72(m, 2H), 7.60-7.58(m, 2H), 7.53-7.5(m, 2H), 3.69(d, J=4.5Hz, 2H), 2.42(s, 8H), 1.08(s, 6H) |
| 48 | 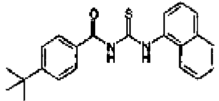 | 363 | ¹H NMR(500MHz, CDCl₃) 12.82(s, 1H), 9.28(s, 1H), 8.08(m, 2H), 7.91(d, J=8.5Hz, 2H), 7.87(d, J=8.0Hz, 1H), 7.62-7.54(m, 6H), 1.40(s, 9H) |
| 49 | 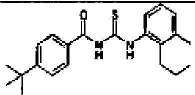 | 367 | |
| 50 | 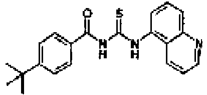 | 364 | ¹H NMR(500MHz, CDCl₃) 12.85(s, 1H), 9.31(s, 1H), 9.01-9.00(m, 1H), 8.45(d, J=8.5Hz, 1H), 8.21(d, J=8.5Hz, 1H), 8.11(d, J=7.5Hz, 1H), 7.91(d, J=8.5Hz, 2H), 7.84(t, J=8.3Hz, 1H), 7.81(d, J=8.5Hz, 1H), 7.57-7.54(m, 1H), 1.40(s, 9H) |
| 51 | 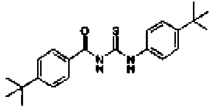 | 369 | ¹H NMR(500MHz, CDCl₃) 12.59(s, 1H), 9.07(s, 1H), 7.84(d, J=8.5Hz, 2H), 7.66(d, J=8.5Hz, 2H), 7.57(d, J=8.5Hz, 2H), 7.45(d, J=8.5Hz, 2H), 1.38(s, 9H), 1.35(s, 9H) |
| 52 | 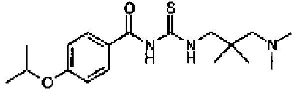 | 352 | ¹H NMR(500MHz, CDCl₃) 11.51(s, 1H), 8.92(s, 1H), 7.81(d, J=9.0Hz, 2H), 6.95(d, J=9.0Hs, 2H), 4.68-4.64(m, 1H), 2.41(s, 8H), 1.38(d, J=6.0Hz, 6H), 1.07(s, 6H) |
| 53 | 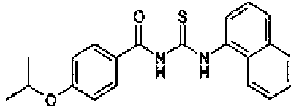 | 365 | ¹H NMR(500MHz, CDCl₃) 12.86(s, 1H), 9.22(s, 1H), 8.05(d, J=8.0Hz, 2H), 7.93(d, J=8.5Hz, 3H), 7.86(d, J=8.0Hz, 1H), 7.61-7.54(m, 3H), 7.02(d, J=9.0Hz, 2H), 4.72-4.69(m, 1H), 1.43-1.41(m, 6H) |
| 54 | 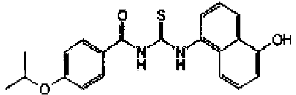 | 381 | ¹H NMR(500MHz, CDCl₃) 12.83(s, 1H), 9.24(s, 1H), 8.30(d, J=8.5Hz, 1H), 8.06(d, J=7.5Hz, 1H), 7.93(d, J=9.0Hz, 2H). 7.61(d, 8.5Hz, 1H), 7.5(t, J=8.0Hz, 1H), 7.39(t, J=8.0Hz, 1H), 7.07-7.00(m, 3H), 6.89(d, 7.5Hz, 1H), 4.71-4.68(m, 1H), 1.41(s, 6H) |
| 55 | 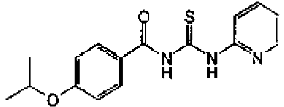 | 316 | ¹H NMR(500MHz, CDCl₃) 13.32(s, 1H), 9.08(s, 1H), 8.83(d, J=8.5Hz, 1H), 8.45(d, J=4.0Hz, 1H), 7.89(d, J=8.5Hz. 2H), 7.82(t, J=7.7Hz, 1H), 7.20(t, J=6.0Hz, 1H), 6.99(d, J=8.5Hz, 2H), 4.68(t, J=6.0Hz, 1H), 1.40(d, J=5.5Hz, 9H) |
| 56 | 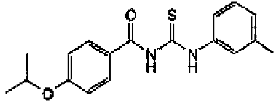 | 329 | ¹H NMR(500MHz, CDCl₃) 12.63(s, 1H), 9.01(s, 1H), 7.87-7.85(m, 2H), 7.55(d, J=8.0Hz, 1H), 7.51(s, 1H), 7.32(t, J=7.7Hz, 1H), 7.11(d, J=7.0Hz, 1H), 7.01-6.98(m, 2H), 4.70-4.67(m, 1H), 2.44(s, 3H), 1.41-1.40(m, 6H) |
| 57 | 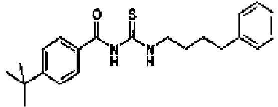 | 369 | |
| 58 | 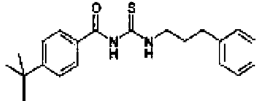 | 355 | |
| 59 | 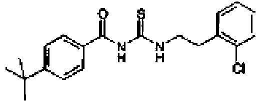 | 375 | |
| 60 | 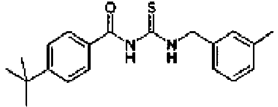 | 341 | ¹H NMR(CDCl₃, 500MHz) 11.04(s, 1H), 10.28(s, 1H), 9.08-9.03(m, 1H), 8.19(d, J=8.5Hz, 1H), 7.83-7.77(m, 1H), 7.54-7.44(m, 2H), 7.29-7.10(m, 3H), 4.04(s, 2H), 2.34(s, 3H), 1.36(s, 9H) |
| 61 | 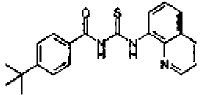 | 364 | |
| 62 | 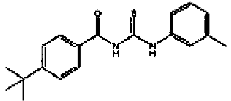 | 327 | ¹H NMR(DM50, 500MHz) : 12.67(s, 1H), 11.45(s, 1H), 7.95(d, J=8.4Hz, 2H), 7.56(d, 1=8.4 Hz, 2H), 7.53(d, J=10Hz, 1H), 7.49(s, 1H), 7.31(t, J=5.3Hz, 1H), 7.1(d, J=2.5, 1H) 2.33(s, 3H), 1.31(s, 9H) |
| 63 | 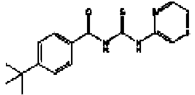 | 315 | ¹H NMR(CDCl₃, 500MHz) 13.21(s, 1H), 10.11(s, 1H), 9.33(s, 1H), 9.15(s, 1H), 8.48-8.40(m, 2H), 7.87(d, J=8.5Hz, 1H), 7.63-7.54(m, 2H), 1.38(z, 9H) |
| 64 | 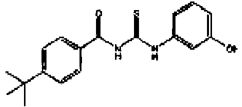 | 329 | ¹H NMR(CDCl₃, 500MHz) : 12.7(s, 1H), 9.05(s,1H), 7.84(d, J=8.4Hz, 2H), 7.57(d, J=8.4Hz, 2H), 7.5B(m, 1H), 7.30-7.27(m, 1H), 7.19(d, J=7.9 1H), 6.78-8.39(m, 1H), 1.37(s,9H) |
| 65 | 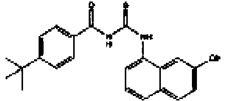 | 379 | ¹H NMR(CDCl₃, 500MHz) 12.64(s, 1H), 9.28(s, 1H), 7.96(d, J=7.5Hz, 1H), 7.87-7.77(m, 4H), 7.56(d, J=8.0Hz, 2H), 7.40(t, J=7.7Hz, 1H), 7.35(s, 1H), 7.18-7.16(m, 1H), 1.39(s, 9H) |
| 66 | 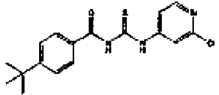 | 348 | ¹H NMR(CDCl₃, 500MHz) 13.18(s, 1H), 9.13(s, 1H). 8.39(d, J=5.0Hz, 1H), 8.13(s, 1H), 7.84(d, J=9.0Hz, 2H). 7.76(d, J=6.0Hz, 1H), 7.58(d, J=8.5Hz, 2H), 1.37(s, 9H) |
| 67 | 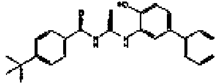 | 415 | |
| 68 | 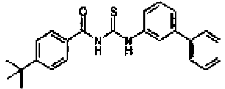 | 389 | |
| 69 | 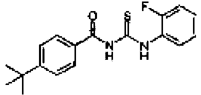 | 331 | ¹H NMR(DMSO, 500MHz) : 12.63(s, 1H), 11.67(s, 1H), 8.04(t, J=7.8Hz, 1H), 7.96(d, J=8.6Hz, 2H), 7.36-7.33(m, 1H), 7.27-7.24(m, 1H), 1.32(s, 9H) |
| 70 | 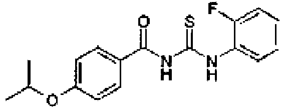 | 333 | ¹H NMR(CDCl₃, 500MHz) : 12.80(s, 1H), 9.07(s, 1H), 8.42(t, J=4.0Hz, 1H), 7.81(d, J=5.0Hz, 2H), 7.18-7.28(m, 3H), 7.00(d, J=3.0Hz, 2H), 4.66-4.72(m, 1H), 1.41(s, 6H) |
| 71 | 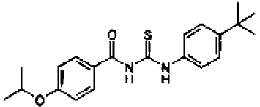 | 371 | ¹H NMR(CDCl₃, 500MHz) ; 12.63(s, 1H), 8.99(s, 1H), 7.85(d, J=4.5Hz, 2H), 7.64(d, J=4.5Hz, 2H), 7.45(d, J=5.0Hz, 2H), 6.99(d, J=5.5, 2H), 4.684(m, 1H), 1.40(s, 6H), 1.35(s, 9H) |
| 72 | 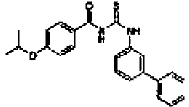 | 391 | ¹H NMR(CDCl₃, 500MHz) : 12.80(s, 1H), 9.04(s, 1H), 7.99-8.00(m, 1H), 7.86-7.88(m, 2H), 7.12-7.74(m, 1H), 7.62-7.6(m, 2H), 7.46-7.54(m, 4H), 7.37-7.40(m, 1H), 6.99-7.00(m, 2H), 4.68-4.70(m, 1H), 1.40(s, 6H) |
| 73 | 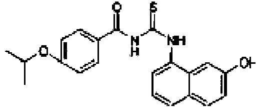 | 381 | ¹H NMR(CDCl₃, 500MHz) : 12.68(s, 1H), 9.21(s, 1H), 7.95(d, J=7.5Hz, 1H), 7.89(d, J=1.5Hz, 2H), 7.85(d, J=12.0Hz, 1H), 7.61(d, J=12.0Hz, 1H), 7.41(t, J=7.7Hz, 1H), 7.33(d, J=2.5Hz, 1H), 7.13-7.18(m, 1H), 7.00(d, J=9.0Hz, 2H), 5.59(s, 1H), 4.69-4.71(m, 1H), 1.42(s, 6H) |

### EXAMPLE 74: Preparation of 1-(4-tert-Butyl-benzyl)-3-(5-hydroxy-naphthalen-1-yl)-thiourea According to Reaction Scheme 2

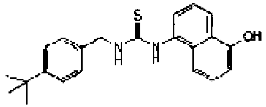

At room temperature, 5-amino-naphthalen-1-ol (0.5 g, 3.14 mmol) was mixed with methylene chloride (31.4 mL, 0.1 M) and sodium hydrogen carbonate (2.64 g, 31.4 mmol) at 0°C for 15 min with stirring. To the reaction mixture was added thiophosgene (1.20 mL, 15.71 mmol), followed by stirring for 2 hrs. The resulting mixture was mixed at room temperature for 6 hrs with 4-tert-butyl-benzylamine (0.60 mL, 3.45 mmol) with stirring. After concentration in vacuo, purification by silica gel column chromatography afforded the title compound (65 mg, Yield: 5.73 %).
¹H NMR (500MHz, CDCl₃): 12.92(s, 1H), 10.59(s, 1H), 7.65(d, J=8.5Hz, 1H), 7.48-7.45(m, 2H), 7.36-7.32(m, 2H), 7.23(s, 1H), 7.17(d, J=8.0Hz, 1H), 6.89(s, 1H), 6.84(d, J=8.0Hz, 1H), 6.69(s, 1H), 4.63(s, 1H), 4.56(s, 2H), 1.33(s, 9H)
LC-MS (M⁺H) : 365

### EXAMPLE 75: Preparation of 1-(4-tert-Butyl-benzyl)-3-(5-hydroxy-naphthalen-1-yl)-urea According to Reaction Scheme 3

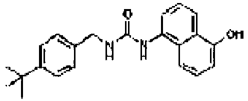

At room temperature, methylene chloride (31.4 mL, 0.1 M) and sodium hydrogen carbonate (2.64 g, 31.4 mmol) were added to 5-amino-naphthalen-1-ol (0.5 g, 3.14 mmol), and the mixture was stirred at 0°C for 15 min. The reaction mixture was mixed with phosgene (8.26 mL, 15.71 mmol) for 2 hrs with stirring. Subsequently, 4-tert-butyl-benzylamine (0.60 mL, 3.45 mmol) was added and stirred at room temperature for 6 hrs. After concentration in vacuo, purification by silica gel column chromatography afforded the title compound (157 mg, Yield: 14.33%)
¹H NMR (500MHz, CDCl3): 8.17(d, J=9.0Hz, 1H), 7.53(s, 2H), 7.48(s, 1H), 7.37-7.32(m, 2H), 7.17(d, J=7.5Hz, 2H), 6.88(d, J=7.0Hz, 1H), 4.43(s, 2H), 4.14(d, J=7.5Hz, 2H), 1.37-1.26(m, 9H)
LC-MS(M⁺H) : 349

### EXAMPLES 76 AND 77

The same procedure as in Example 75 was carried out to prepare compounds of Examples 76 and 77. In Table 2, structural formulas, MS data, and ¹H NMR data of the compounds of Examples 76 and 77 are summarized.

**TABLE 2**

| 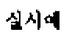 | 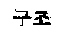 | LC-MS (M⁺H) | ¹H NMR |
|---|---|---|---|
| 76 | 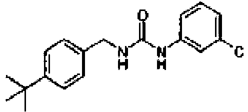 | 317 | |
| 77 | 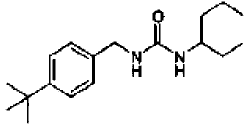 | 289 | |

### EXAMPLE 78: Preparation of 1-(4-tert-Butyl-benzoyl)-3-(5-hydroxy-naphthalen-1-yl)-urea According to Reaction Scheme 4

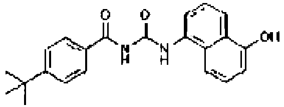

### 1. Synthesis of 4-tert-butyl-benzoamide

At room temperature, tetrahydrofuran (224.4 mL, 0.05 M) and 1,1'-carbonyldiimidazole (2 g, 12.34 mmol) were added to 4-tert-butyl-benzoic acid (2 g, 11.22 mmol) and stirred for 12 hrs. The reaction mixture was mixed at room temperature with 28 % ammonia water (56.1 mL, 0.2 M) and stirred for 4 hrs. After layer division, the organic layer was withdrawn, dried over magnesium sulfate, and distilled in a vacuum. The concentrate was purified by silica gel column chromatography to afford the title compound.

### 2. Synthesis of 1-(4-tert-butyl-benzoyl)-3-(5-hydroxy-naphthalen-1-yl)-urea

At room temperature, methylene chloride (10.46 mL, 0.3 M) and sodium hydrogen carbonate (0.97 g, 31.4 mmol) were added to 5-amino-naphthalen-1-ol (0.5 g, 3.14 mmol) and stirred at 0°C for 15 min. The reaction mixture was mixed with phosgene (8.26 mL, 15.71 mmol) and stirred for 2 hrs. Subsequently, the reaction mixture was allowed to react with 4-tert-butyl-benzoamide (0.61 g, 3.45 mmol), synthesized in 1, at room temperature for 6 hrs with stirring. After concentration in a vacuum, purification by silica gel column chromatography afforded the title compound (120 mg, Yield: 10.51%).
¹H NMR (500 MHz, DMSO): 11.54(s, 1H), 11.14(s, 1H), 10.28(s, 1H), 8.17-7.87(m, 3H), 7.59-7.47(m, 5H), 6.96(s, 1H), 4.14(t, J=6.8Hz, 1H), 1.33(s, 9H)

### EXAMPLE 79: Preparation of 1-(4-tert-Butyl-benzoyl)-3-pyridin-2-yl-urea According to Reaction Scheme 5

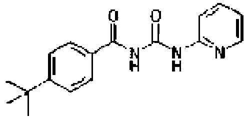

### 1. Synthesis of 4-(tert-butyl)-benzoyl chloride

At room temperature, 4-(tert-butyl)-benzoic acid (2 g, 11.22 mmol) was mixed with methylene chloride (112.2 mL, 0.1 M) and 2 M oxalyl chloride (16.8 mL, 33.6 mmol), and two or three drops of dimethyl formamide were added to the mixture, followed by stirring at 70°C for 6 hrs. concentration in vacuo yielded the title compound.

### 2. Synthesis of 1-(4-tert-butyl-benzoyl)-3-(pyridin-2-yl)-urea

4-(Tert-butyl)-benzoyl chloride, synthesized in 1, was mixed with acetonitrile (22.44 mL, 0.5 M) and toluene (22.44 mL, 0.5 M), and then with 1 M tin chloride (SnCl₄) (0.56 mL, 0.56 mmol), and sodium cyanate (NaOCN) (0.88 g, 13.46 mmol) for 8 hrs under a flux condition. After the distillation of the reaction mixture in a vacuum, the distillate was mixed 2-aminopyridine (1.06 g, 11.22 mmol) for 2 hrs at room temperature by stirring. The organic layer was withdrawn from the aqueous layer, dried over magnesium sulfate, and distilled in vacuo. The crude product was further purified by silica gel column chromatography to afford the title compound (66 mg, Yield: 3.92 %).
¹H NMR (500 MHz, CDCl₃) : 11.36(s, 1H), 8.92(s, 1H), 8.39-8.37(m, 1H), 8.13(d, J=8.0Mz, 1H), 7.93(d, J=9.0Mz, 3H), 7.75-7.71(m, 1H), 7.56-7.54(m, 3H), 7.10-7.08(m, 1H)
LC-MS(M⁺H) : 298

### EXAMPLES 80 TO 84

The same procedure as in Example 78 was carried out to prepare compounds of Examples 80 to 84. In Table 3, structural formulas, MS data, and ¹H NMR data of the compounds of Examples 80 to 84 are summarized.

**TABLE 3**

| 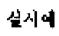 | 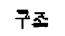 | LC-MS (M⁺H) | ¹H NMR |
|---|---|---|---|
| 80 | 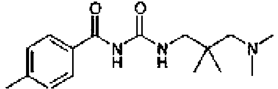 | 292 | ¹H NMR(500MHz, CDCl₃) 9.08(s, 1H), 8.11(s, 1H). 7.74(d, J=8.0Hz, 2H), 7.30(d, J=8.5Hz, 2H), 3.29(d, J=6.0Hz, 2H). 2.34(s, 3H). 2.38-2.31(m, 6H). 2.22(s, 2H), 0.97(s, 6H) |
| 81 | 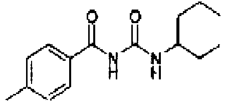 | 283 | ¹H NMR(500MHz, CDCl₃) 8.59(s, 1H), 8.14(s, 1H). 7.074(d, J=7.0Hz, 2H), 7.28(d, J=12.5Hz, 2H). 3.81-3.79(m, 1H). 2.44(s, 3H), 2.00-1.98(m, 2H), 1.75-1.74(m, 2H). 1,61(s, 1H). 1.41-1.25(m, 5H) |
| 82 | 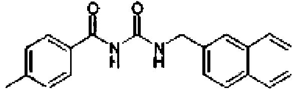 | 341 | ¹H NMR(500MHz, CDCl₃) 10.73(s, 1H). 9.07(s, 1H) |
| 83 | 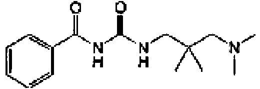 | 278 | ¹H NMR(500MHz, CDCl₃) 9.24(s, 1H), 7.80(d, J=7.0Hz, 2H). 7.52-7.42(m. 3H), 3.39(d, J=4.5Hz, 2H). 2.38(s, 8H). 1.01(s, 6H) |
| 84 | 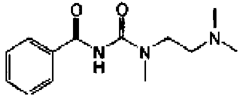 | 250 | |

### EXAMPLES 85∼99:

The same procedure as in Example 1 (Reaction Scheme 1) was carried out to prepare compounds of Examples 85 to 99. In Table 4, structural formulas, MS data, and ¹H NMR data of the compounds of Examples 85 to 99 are summarized.

**TABLE 4**

| 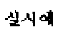 | 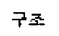 | LC-MS (M'H) | ¹H NMR |
|---|---|---|---|
| 85 | 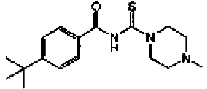 | 320 | ¹H NMR(500MHz, CDCl₃) 8.55(s, 1H), 7.88(d. J=8.5Hz, 2H). 7.60(d, J=8.5Hz, 2H), 4.41(s, 2H), 3.82(s, 2H), 2.79(s. 3H), 2.52(s, 4H), 1.44(s, 9H) |
| 86 | 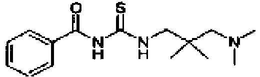 | 294 | ¹H NMR(500MHz, CDCl₃) 11.66(s, 1H), 9.05(s, 1H), 7.86(d, J=7.0Hz, 2H). 7.61(t, J=7.5Hz, 1H). 7.50(t, J=7.7Hz, 2H). 3.64(d, J=4.5Hz, 2H). 2.32(d, J=21.5Hz, 8H). 1.02(s, 6H) |
| 87 | 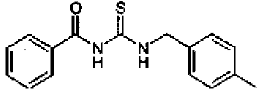 | 285 | ¹H NMR(500MHz, CDCl₃) 10.89(s. 1H), 8.95(s. 1H), 7.75(d, J=7.0Hz, 2H). 7.55(t, J=7.5Hz, 1H). 7.44(t, J=7.7Hz, 2H). 7.20(t, J=8.2Hz, 2H), 7.11(d, J=8.0Hz, 2H), 4.80(d, J=5.5Hz, 2H), 2.28(s, 3H) |
| 88 | 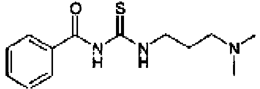 | 266 | |
| 89 | 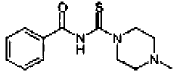 | 278 | ¹H MMR(500MHz. CDCl₃) 8.36(s, 1H), 7.79-7.76(m, 2H), 7.55-7.51(m, 1H), 7.44-7.41(m, 2H). 4.20(s. 2H). 3.62(s, 2H), 2.55(d, J=30Hz, 4H). 2.44-2.40(m, 2H), 1.05(t, J=7.3Hz, 3H) |
| 90 | 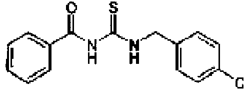 | 305 | ¹H NMR(500MHz, CDCl₃) 7.76(d, J=8.0Hz, 2H). 7.57(t, J=7.5Hz, 1H). 7.46(t. J=7.7Hz, 2H). 7.29-7.25(m. 3H). 7.19(s, 2H). 4.83(d, J=5.5Hz, 2H) |
| 91 | 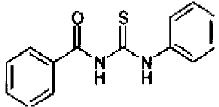 | 257 | ¹H NMR(500MHz, CDCl₃) 12.60(s, 1H), 9.09(s, 1H), 7.93-7.91(m, 2H). 7.75-7.73(m, 2H). 7.70-7.67(m. 1H), 7.59-7.56(m, 2H), 7.47-7.43(m, 2H), 7.33-7.28(m, 1H) |
| 92 | 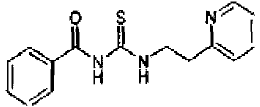 | 286 | ¹H NMR(500MHz, CDCl₃) 11.11(s. 11H), 8.95(s, 1H), 8.64-8.63(m, 1H). 7.83(d, J=8.0Hz, 2H). 7.67-7.61(m, 2H), 7.53-7.50(m, 2H). 7.25-7.24(m. 1H), 7.20-7.18(m, 1H). 4.20-4.17(m, 2H). 3.33(t. J=6.5Hz, 2H) |
| 93 | 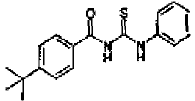 | 313 | ¹H NMR(500MHz, CDCl₃) 12.66(s. 1H). 9.07(s. 1H). 7.85(d, J=8.5Hz, 2H), 7.74(d, J=7.5Hz, 2H). 7.57(d, J=9.0Hz. 2H), 7.44(t, J=7.7Hz, 2H), 7.30(t, J=7.5Hz, 2H), 1.38(s, 9H) |
| 94 | 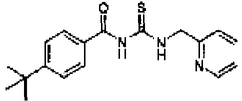 | 328 | ¹H MMR(500MHz, CDCl₃) 11.70(s, 1H), 9.038(s, 1H). 8.68(d. J=4.5Hz. 1H), 7.83(d, J=8.5Hz, 2H), 7.74-7.70(m, 1H). 7.54(d. J=8.5Hz, 2H), 7.35(d, J=7.5Hz. 1H), 7.26-7.25(m, 1H). 5.05(d, J=4.5Hz, 2H). 1.37(s, 9H) |
| 95 | 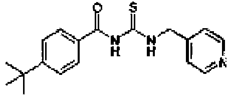 | 328 | |
| 96 | 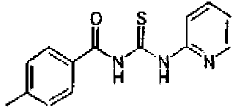 | 272 | ¹H NMR(500MHz, CDCl₃) 13.17(s,1H), 9.02(s. 1H). 8.84(d, J=8.5Hz, 1H), 8.47-8.46(m, 1H), 7.83-7.78(m, 3H), 7.36(d, J=8.0Hz, 2H). 7.20.7.17(m, 1H), 2.47(s, 3H) |
| 97 | 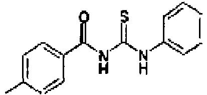 | 271 | ¹H NMR(500MHz, CDCl₃ 12.65(s, 1H), 9.07(s, 1H). 7.80(s, 2H), 7.73(d, J=7.5Hz, 2H). 7.44-7.27(m, 5H), 2.47(s. 3H) |
| 98 | 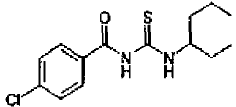 | 297 | ¹H NMR(500MHz, CDCl₃) 10.65(s, 1H), 8.86(s, 1H). 7.79(d. J=13.0Hz, 2H). 7.51(d. J=11.0Hz, 2H). 4.32-4.28(m. 1H). 2.12-2.10(m, 2H). 1.78-1.75(m, 2H). 1.51-1.38(m, 5H), 1.35-1.27(m,1H) |
| 99 | 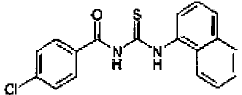 | 341 | ¹H NMR(500MHz, CDCl₃) 12.68(s, 1H). 9.26(s. 1H). 8.05-8.03(m, 2H). 7.95-7.91(m, 3H), 7.88(d, J=8.0Hz, 1H), 7.62-7.55(m, 5H) |

### EXAMPLE 100

The same procedure as in Example 78 (Reaction Scheme 4) was carried out to prepare the compound of Example 94. In Table 5, the structural formula, MS data, and ¹H NMR data of the compound of Example 100 are given.

**TABLE 5**

| 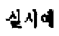 | 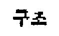 | LC-MS (M⁺H) | ¹H NMR |
|---|---|---|---|
| 100 | 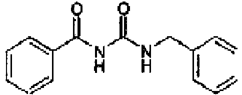 | 277 | ¹H NMR(500MHz, CDCl₃) 9.05(s, 1H), 8.70(s, 1H), 7.89(s, 1H), 7.82(s, 2H), 7.62-7.46(m, 6H), 7.33-7.28(m, 2H). 4.68(s, 2H) |

### EXPERIMENTAL EXAMPLE 1: Assay for Inhibitory Activity of the Thiourea or Urea Derivatives against HIV

The thiourea or urea derivatives of the present invention were assayed for inhibitory activity against HIV as follows.

### 1. Preparation of HIV

A pNL4-3 vector carrying an HIV genome was transduced into 293FT cells. The HIV genome on pNL4-3 does not infect the body because it contains an EGFP gene in substitution for the nef. 293FT cells were seeded at a density of 3.0×10⁵ cells/well into 6-well plates containing antibiotic-free DMEM supplemented with 10 % FBS and grown for 48 hrs. Upon 90 ∼ 95 % confluency, the transduction was performed.

The DNA (pNL4-3) was added at a final concentration of 10 ng/mL to 250 µL of the reduced serum medium Opti-MEM I (GIBCO™, USA). Separately, 4 µL of Lipofectamine™ 2000 (Invitrogen, USA) was mixed with 250 µL of Opti-MEM I (GIBCO™), and incubated at room temperature for 5 min. The DNA (pNL4-3) was mixed at a ratio of 1:2 with the lipofectamine and reacted at room temperature for 20 min to form a DNA-lipofectamine complex. After removal of the medium from the cell culture, the DNA-lipofectamine complex was added to the cells and incubated for 4 hrs in a CO₂ incubator. Then, the cell culture medium was replaced with a fresh antibiotic-free, 10 % FBS-supplemented DMEM, followed by incubation for an additional 48 hrs. The HIV-infected cell culture was centrifuged at 1,500 rpm for 5 min to give a virus supernatant.

MT-4 cells were plated at a density of 1.0×10⁶ cells/mL into 6-well plates containing RPMI supplemented with 10 % FBS, and incubated for 4 hrs before infection at a ratio of 1:1 with the virus supernatant. The cells were incubated for 48 hrs in a CO₂ incubator, after which the culture medium was replaced with a fresh one. Again, incubation was continued for an additional 72 hrs to amplify the virus. After centrifugation at 1,500 rpm for 5 min, the virus supernatant thus obtained was examined for virus density using a Virononstika HIV-1 Antigen Microelisa system kit (bioMerieux, Inc. Netherland).

### 2. Assay for Inhibitory Activity against HIV

The thiourea or urea derivatives prepared in Examples 1 to 94 were dissolved in DMSO to prepare respective 10 mM or 20 mM stocks, and stored at -20°C. The stocks were diluted to a starting concentration of 200 µM in DMSO before use in experiments.

MT-4 cells were suspended at a density of 4×10⁵ cells in 200 µL of RPMI 1640 (Gibco, 10 % FBS) per well of 24-well plates, and infected with 200 µL of a virus supernatant containing 2x10⁵ pg/mL to give a total volume of 400 µL. After the stocks of the thiourea or urea derivatives were 10-fold diluted to 200 µM in DMSO, 2 µL of each dilution was added to the cells to a final concentration of 1 µM or less. For a positive control, 10 µM AZT (azidothymidine, Sigma, USA) was used at a final concentration of 50 nM while DMSO was used at a final concentration of 0.5 % as a negative control. The cells were cultured for 48 hrs in a CO₂ incubator (37°C, 5 % CO₂), and the culture medium was replaced by RPMI 1640 containing the thiourea or urea derivative at a final concentration of 1 µM, followed by incubation for an additional 48 hrs. Virus soups were separated from cells by centrifugation at 1,500 rpm for 5 min and then at 13, 000 rpm for 5 min. ELISA for p24 concentrations was carried out using a Virononstika HIV-1 Antigen Microelisa system kit (bioMerieux, Inc. Netherland) to determine the inhibitory activity of each of the thiourea or urea derivatives against HIV.

Anti-HIV activities (µM) of the thiourea or urea derivatives of the present invention are summarized in Table 6, below.

**TABLE 6**

| Example No. | Conc. for Inhibition of HIV Activity (µM) |
|---|---|
| 1 | 0.03 |
| 16 | 0.5 |
| 37 | 0.5 |
| 38 | 0.3 |
| 50 | 1.0 |
| Positive Control (AZT) | 0.005 |

As can be seen in Table 6, the thiourea or urea derivatives of the present invention exhibited high inhibitory activity against HIV.

Formulation examples are given to illustrate dosage preparations containing the composition of the present invention.

### FORMULATION EXAMPLE 1: Preparation of Powder

Thiourea or Urea Derivative of the present invention 0.1 g
Lactose 1.5 g
Talc 0.5 g

These ingredients were mixed and loaded into an airtight sac to give a powder.

### FORMULATION EXAMPLE 2: Preparation of Tablet

Thiourea or Urea Derivative of the present invention 0.1 g
Lactose 7.9 g
Crystalline cellulose 1.5 g
Magnesium stearate 0.5 g

These ingredients were mixed and directly compressed into a tablet.

### FORMULATION EXAMPLE 3: Preparation of Capsule

Thiourea or Urea Derivative of the present invention 0.1 g
Corn starch 5 g
Carboxycellulose 4.9 g

These ingredients were admixed together and the admixture was loaded into a conventional capsule using a suitable device.

### FORMULATION EXAMPLE 4: Preparation of Injection

Thiourea or Urea Derivative of the present invention 0.1 g
Sterile water for injection suitable amount
pH Adjuster suitable amount
Stabilizer suitable amount

Using a conventional method, these ingredients were put into an ampule (2 ml) to give an injection.

### FORMULATION EXAMPLE 5: Preparation of Liquid Medicine

Thiourea or Urea Derivative of the present invention 0.1 g
Isomerized sugar 10 g
Mannitol 5 g
Purified water suitable amount

Each ingredient was dissolved in purified water and flavored with lemon before admixing together. Purified water was added to the admixture to form a final volume of 100 ml which was then loaded into a brown vial and sterilized.

### Industrial Applicability

As described hitherto, the thiourea or urea derivatives of the present invention inhibit HIV virus expression, thus finding applications in the prevention or treatment of AIDS.

## Claims

1. A thiourea or urea derivative, represented by the following Chemical Formula 1, or a pharmaceutically acceptable salt thereof: 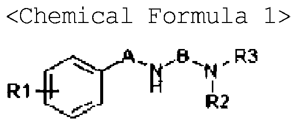 wherein,
A represents -CH₂ or -C(=O),
B represents -C(=O) or -C(=S),
R1 represents H, a halogen atom, linear or branched alkyl of C1∼C4, phenyl, phenyl substituted with linear or branched alkyl of C1∼C4, linear or branched alkoxy of C1∼C4, aryloxy of C6∼C20, or 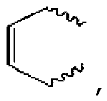 R2 and R3, which may be the same or different, independently represent H, linear or branched alkyl of C1∼C4, cycloalkyl of C3∼C8, -(linear or branched alkyl of C2∼C6)-N-(alkyl of C1∼C2)₂, -(linear or branched alkyl of C1∼C4)-NH-(linear or branched alkyl of C1∼C4), 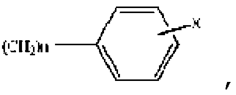 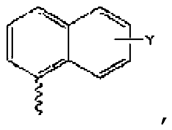 5,6,7,8-tetrahydronaphthalen-1-yl, (naphthalen-2-yl)-methyl, 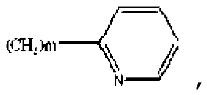 (pyridin-3-yl)-methyl, 2-chloro-pyridin-4-yl, pyrazin-2-yl, quinolin-5-yl, quinolin-8-yl, or 4-hydroxy-biphenyl-3-yl, or R2 and R3 are fused to each other, forming 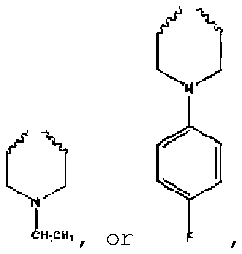 n is an integer of 0, or 1 to 4,
m is an integer of 0, 1 or 2,
X represents H, linear or branched alkyl of C1∼C4, aryl of C6∼C20, hydroxy, or a halogen atom, and
Y represents H or hydroxy,
with the provisos of the following conditions:
when A, B, and R1 represent -C(=O), -C(=S), and H,
respectively, to the exclusion of the options that R2 represents H, and R3 represents -CH₂-C(CH₃)₂-CH₂-N-(CH₃)₂, - (CH₂)₃-N-(CH₃)₂, phenyl, 4-methyl-benzyl, 4-chloro-benzyl, or (pyridin-2-yl)-ethyl, or R2 does not represent H, excluding the option that R2 and R3 are fused to each other, forming 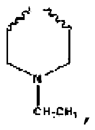 when A, B, and R1 represent -C(=O), -C(=S), and tert-butyl, respectively, to the exclusion of the option that R2 is H, and R3 represents phenyl, naphthalene, 3-chloro-phenyl, 3-toyl, 2-fluoro-phenyl or (pyridin-2-yl)-methyl,
when A, B, and R1 represent -C(=O), -C(=S), and methyl, respectively, to the exclusion of the option that R2 represents H, and R3 represents phenyl, naphthalene or pyridin-2-yl,
when A, B, and R1 represent -C(=O), -C(=S), and Cl, respectively, to the exclusion of the option that R2 represents H, and R3 represents cyclohexyl or naphthalene,
when A, B, and R1 represent -C(=O), -C(=O), and H, respectively, to the exclusion of the option that R2 represents H, and R3 represents benzyl.

2. The thiourea or urea derivative, or the pharmaceutically acceptable salt of claim 1, wherein
R1 represents H, Cl, methyl, tert-butyl, phenyl, (tert-butyl)-phenyl, isopropoxy, phenoxy, or 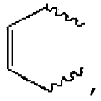 R2 and R3, which may be the same or different, independently represent H, methyl, cyclohexyl, -CH₂CH₂-N-(CH₃)₂. -CH₂CH₂CH₂-N- (CH₃)₂, -CH₂-C(CH₃)₂-CH₂-N-(CH₃)₂, -CH₂CH₂-N-(CH₂CH₃)₂, -CH (CH₃) CH₂CH₂CH₂-N-(CH₂CH₃)₂, -(CH₂)₃-NH-(CH₂CH₂CH₃), 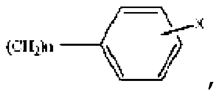 naphthalen-1-yl, 5-hydroxy-naphthalen-1-yl, 7-hydroxy-naphthalen-1-yl, 5,6,7,8-tetrahydronaphthalen-1-yl, pyridin-2-yl, (pyridin-2-yl)-methyl, (pyridin-2-yl)-ethyl, (pyridin-3-yl)-methyl, 2-chloro-pyridin-4-yl, pyrazin-2-yl, quinolin-5-yl, quinolin-8-yl, or 4-hydroxy-biphenyl-3-yl, or R2 and R3 are fused to each other, forming 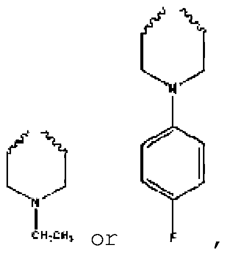 n is an integer of 0, or 1 to 4, and
X represents H, methyl, tert-butyl, phenyl, hydroxy, F, Cl, or Br.

3. The thiourea or urea derivative, or the pharmaceutically acceptable salt of claim 1, wherein the thiourea or urea derivative is selected from the group consisting of:
1) 1-(4-tert-butyl-benzoyl)-3-(5-hydroxy-naphthalen-1-yl)-thiourea,
2) 1-(4-tert-butyl-benzoyl)-3-(3-dimethylamino-propyl)-thiourea,
3) 1-(4-tert-butyl-benzoyl)-3-(3-dimethylamino-2,2-dimethyl-propyl)-thiourea,
4) 4-tert-butyl-N-(4-ethyl-piperazin-1-carbothioyl)-benzoamide,
5) 3-(4-tert-butyl-benzoyl)-1-(2-diethylamino-ethyl)-1-methyl-thiourea,
6) 3-(4-tert-butyl-benzoyl)-1-(2-dimethylamino-ethyl)-1-methyl-thiourea,
7) 1-(4-tert-butyl-benzoyl)-3-(4-diethylamino-1-methylbutyl)-thiourea,
8) 1-(4-tert-butyl-benzoyl)-3-(4-methyl-benzyl)-thiourea,
9) 1-(4-tert-butyl-benzoyl)-3-(2-methyl-benzyl)-thiourea,
10) 1-(4-tert-butyl-benzoyl)-3-(3-propylamino-propyl)-thiourea,
11) 1-(4-tert-butyl-benzoyl)-3-phenethyl-thiourea,
12) 1-(4-tert-butyl-benzoyl)-3-(4-chloro-benzyl)-thiourea,
13) 1-(4-tert-butyl-benzoyl)-3-(3-chloro-benzyl)-thiourea,
14) 1-(4-tert-butyl-benzoyl)-3-(naphthalen-2-ylmethyl)-thiourea,
15) 4-tert-butyl-N-[4-(4-fluoro-phenyl)-piperazin-1-carbothioyl]-benzamide,
16) 1-(4-tert-butyl-benzoyl)-3-cyclohexyl-thiourea,
17) 1-(4-tert-butyl-benzoyl)-3-(2-fluoro-benzyl)-thiourea,
18) 1-benzoyl-3-(naphthalen-2-ylmethyl)-thiourea,
19) 1-benzoyl-3-[2-(4-chloro-phenyl)-ethyl]-thiourea,
20) 1-benzoyl-3-(4-diethylamino-1-methyl-butyl)-thiourea,
21) N-[4-(4-fluoro-phenyl)-piperazin-1-carbothioyl]-benzamide,
22) 3-benzoyl-1-(2-diethylamino-ethyl)-1-methyl-thiourea,
23) N-(4-ethyl-piperazin-1-carbothioyl)-4-methyl-benzamide,
24) 1-cyclohexyl-3-(4-methyl-benzoyl)-thiourea,
25) 1-(4-chloro-benzyl)-3-(4-methyl-benzoyl)-thiourea,
26) 1-(3-dimethylamino-propyl)-3-(4-methyl-benzoyl)-thiourea,
27) 1-(4-methyl-benzoyl)-3-(3-methyl-benzyl)-thiourea,
28) 1-(4-methyl-benzoyl)-3-(4-phenyl-butyl)-thiourea,
29) 1-(4-methyl-benzoyl)-3-(3-phenyl-propyl)-thiourea,
30) 1-(3-dimethylamino-2,2-dimethyl-propyl)-3-(4-methylbenzoyl)-thiourea,
31) 1-benzoyl-3-[2-(4-fluoro-phenyl)-ethyl]-thiourea,
32) 1-(4-methyl-benzoyl)-3-(pyridin-3-ylmethyl)-thiourea,
33) 1-(4-methyl-benzoyl)-3-(pyridin-2-ylmethyl)-thiourea,
34) 1-[2-(3-bromo-phenyl)-ethyl]-3-(4-methyl-benzoyl)-thiourea,
35) 1-(4-methyl-benzoyl)-3-(2-pyridin-2-yl-ethyl)-thiourea,
36) 1-(4-tert-butyl-benzoyl)-3-(naphthalen-1-yl)-thiourea,
37) 1-(4-tert-butyl-benzoyl)-3-(pyridin-2-yl)-thiourea,
38) 1-(4-tert-butyl-benzoyl)-3-(3-chloro-phenyl)-thiourea,
39) 1-(4-methyl-benzoyl)-3-(naphthalen-1-yl)-thiourea,
40) 1-(4-chloro-benzoyl)-3-(3-dimethylamino-2,2-dimethylpropyl)-thiourea,
41) 1-(4-chloro-benzoyl)-3-(naphthalen-2-ylmethyl)-thiourea,
42) 1-(4-chloro-benzoyl)-3-(5-hydroxy-naphthalen-1-yl)-thiourea,
43) 1-(4-phenoxy-benzoyl)-3-(pyridin-2-yl)-thiourea,
44) 1-(biphenyl-4-carbonyl)-3-(3-dimethylamino-2,2-dimethyl-propyl)-thiourea,
45) 1-(4'-tert-butyl-biphenyl-4-carbonyl)-3-(3-chloro-phenyl)-thiourea,
46) 1-(3-dimethylamino-2,2-dimethyl-propyl)-3-(naphthalene-2-carbonyl)-thiourea,
47) 1-(biphenyl-4-carbonyl)-3-(3-chloro-phenyl)-thiourea,
48) 1-(4-tert-butyl-benzoyl)-3-(naphthalen-1-yl)-thiourea,
49) 1-(4-tert-butyl-benzoyl)-3-(5,6,7,8-tetrahydro-naphthalen-1-yl)-thiourea,
50) 1-(4-tert-butyl-benzoyl)-3-(quinolin-5-yl)-thiourea,
51) 1-(4-tert-butyl-benzoyl)-3-(4-tert-butyl-phenyl)-thiourea,
52) 1-(3-dimethylamino-2,2-dimethyl-propyl)-3-(4-isopropoxy-benzoyl)-thiourea,
53) 1-(4-isopropoxy-benzoyl)-3-(naphthalen-1-yl)-thiourea,
54) 1-(5-hydroxy-naphthalen-1-yl)-3-(4-isopropoxy-benzoyl)-thiourea,
55) 1-(4-isopropoxy-benzoyl)-3-(pyridin-2-yl)-thiourea,
56) 1-(4-isopropoxy-benzoyl)-3-(m-tolyl)-thiourea,
57) 1-(4-tert-butyl-benzoyl)-3-(4-phenyl-butyl)-thiourea,
58) 1-(4-tert-butyl-benzoyl)-3-(4-phenyl-propyl)-thiourea,
59) 1-(4-tert-butyl-benzoyl)-3-[2-(2-chloro-phenyl)-ethyl)-thiourea,
60) 1-(4-tert-butyl-benzoyl)-3-(3-methyl-benzyl)-thiourea,
61) 1-(4-tert-butyl-benzoyl)-3-(quinolin-8-yl)-thiourea,
62) 1-(4-tert-butyl-benzoyl)-3-(m-tolyl)-thiourea,
63) 1-(4-tert-butyl-benzoyl)-3-(pyrazin-2-yl)-thiourea,
64) 1-(4-tert-butyl-benzoyl)-3-(3-hydroxy-phenyl)-thiourea,
65) 1-(4-tert-butyl-benzoyl)-3-(7-hydroxy-naphthalen-1-yl)-thiourea,
66) 1-(4-tert-butyl-benzoyl)-3-(2-chloro-pyridin-4-yl)-thiourea,
67) 1-(4-tert-butyl-benzoyl)-3-(4-hydroxy-biphenyl-3-yl)-thiourea,
68) 1-biphenyl-3-yl-3-(4-tert-butyl-benzoyl)-thiourea,
69) 1-(4-tert-butyl-benzoyl)-3-(2-fluoro-phenyl)-thiourea,
70) N-(2-fluorophenylcarbamothioyl)-4-isopropoxybenzamide,
71) N-(4-tert-butylphenylcarbamothioyl)-4-isopropoxybenzamide,
72) N-(biphenyl-3-ylcarbamothioyl)-4-isopropoxybenzamide,
73) N-(7-hydroxynaphthalen-1-ylcarbamothioyl)-4-isopropoxybenzamide,
74) 1-(4-tert-butyl-benzyl)-3-(5-hydroxy-naphthalen-1-yl)-thiourea,
75) 1-(4-tert-butyl-benzyl)-3-(5-hydroxy-naphthalen-1-yl)-urea,
76) 1-(4-tert-butyl-benzyl)-3-(3-chloro-phenyl)-urea,
77) 1-(4-tert-butyl-benzyl)-3-cyclohexyl-urea,
78) 1-(4-tert-butyl-benzoyl)-3-(5-hydroxy-naphthalen-1-yl)-urea,
79) 1-(4-tert-butyl-benzoyl)-3-(pyridin-2-yl)-urea,
80) 1-(3-dimethylamino-2,2-dimethyl-propyl)-3-(4-methylbenzoyl)-urea,
81) 1-cyclohexyl-3-(4-methyl-benzoyl)-urea,
82) 1-(4-methyl-benzoyl)-3-(naphthalen-2-ylmethyl)-urea,
83) 1-benzoyl-3-(3-dimethylamino-2,2-dimethyl-propyl)-urea, and
84) 3-benzoyl-1-(2-dimethylamino-ethyl)-1-methyl-urea.

4. A method for preparing the compound of claim 1, as illustrated in the following Reaction Scheme 1, comprising:
1) reacting a carboxylic acid compound of Chemical Formula 2 with oxalyl chloride in an organic solvent to synthesize a compound of Chemical Formula 3,
2) reacting the compound of Chemical Formula 3, synthesized in 1), with potassium thiocyanate (KSCN) in an organic solvent to synthesize a compound of Chemical Formula 4, and
3) reacting the compound of Chemical Formula 4, synthesized in 2), with an amine compound of Chemical Formula 4 to afford a compound of Chemical Formula 1-1: 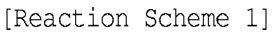 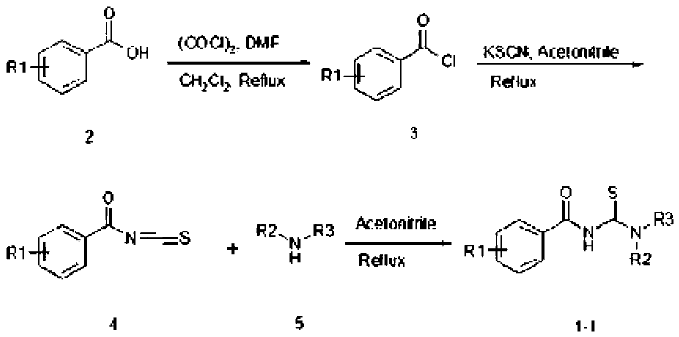 wherein,
R1 represents H, a halogen atom, linear or branched alkyl of C1∼C4, phenyl, phenyl substituted with linear or branched alkyl of C1∼C4, linear or branched alkoxy of C1∼C4, aryloxy of C6∼C20, or 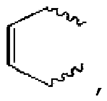 R2 and R3, which may be the same or different, independently represent H, linear or branched alkyl of C1∼C4, cycloalkyl of C3∼C8, -(linear or branched alkyl of C2∼C6)-N-(alkyl of C1∼C2)₂, -(linear or branched alkyl of C1∼C4)-NH-(linear or branched alkyl of C1∼C4), 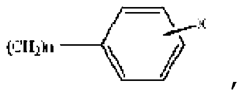 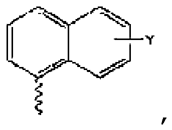 5,6,7,8-tetrahydronaphthalen-1-yl, (naphthalen-2-yl)-methyl, 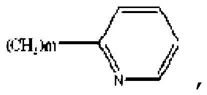 (pyridin-3-yl)-methyl, 2-chloro-pyridin-4-yl, pyrazin-2-yl, quinolin-5-yl, quinolin-8-yl, or 4-hydroxy-biphenyl-3-yl, or R2 and R3 are fused to each other, forming 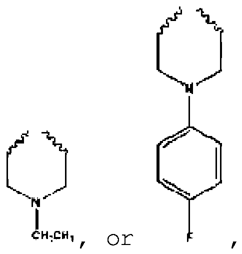 n is an integer of 0, or 1 to 4,
m is an integer of 0, 1 or 2,
X represents H, linear or branched alkyl of C1∼C4, aryl of C6∼C20, hydroxy, or a halogen atom, and
Y represents H or hydroxy,
with a proviso of the following conditions:
when A, B, and R1 represent -C(=O), -C(=S), and H, respectively, ether R2 is H, to the exclusion of the options that R3 represents -CH₂-C(CH₃)₂-CH₂-N-(CH₃)₂, -(CH₂)₃-N-(CH₃)₂, phenyl, 4-methyl-benzyl, 4-chloro-benzyl, or (pyridin-2-yl)-ethyl, or R2 does not represent H, excluding that R2 and R3 are fused to each other, forming 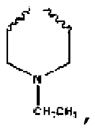
when R1 represent H, either R2 is H, excluding that R3 represents -CH₂-C(CH₃)₂-CH₂-N-(CH₃)₂, -(CH₂)3-N-(CH₃)₂, phenyl, 4-methyl-benzyl, 4-chloro-benzyl, or (pyridin-2-yl)-ethyl, or R2 is not H, excluding that R2 and R3 are fused to each other, forming 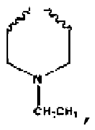
when R1 represents tert-butyl, R2 is H, excluding that R3 represents phenyl or (pyridin-2-yl)-methyl,
when R1 represents methyl, R2 is H, excluding that R3 represents phenyl or pyridin-2-yl, and
when R1 represents Cl, R2 is H, excluding that R3 represents cyclohexyl or naphthalene.

5. A method for preparing the compound of claim 1, as illustrated in the following Reaction Scheme 2, comprising mixing an amine compound of Chemical Formula 5 with sodium hydrogen carbonate in an organic solvent by stirring, and reacting the mixture with thiophosgen and an amine compound of Chemical Formula 6 to afford a compound of Chemical Formula 1-2: 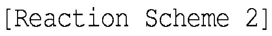 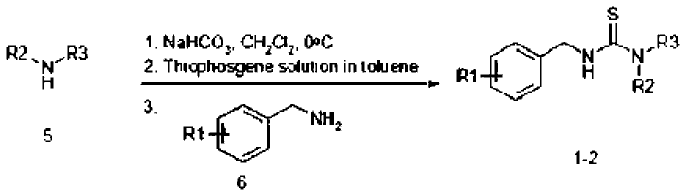 wherein, R1 represents H, a halogen atom, linear or branched alkyl of C1∼C4, phenyl, or phenyl substituted with linear or branched alkyl of C1∼C4, linear or branched alkoxy of C1∼C4, aryloxy of C6∼C20, or 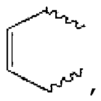 R2 and R3, which may be the same or different, independently represent H, linear or branched alkyl of C1∼C4, cycloalkyl of C3∼C8, -(linear or branched alkyl of C2∼C6)-N-(alkyl of C1∼C2)₂, -(linear or branched alkyl of C1∼C4)-NH-(linear or branched alkyl of C1∼C4), 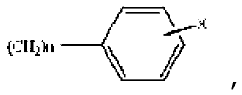 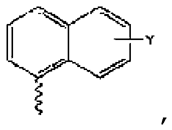 5,6,7,8-tetrahydronaphthalen-1-yl, (naphthalen-2-yl)-methyl, 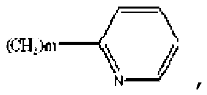 (pyridin-3-yl)-methyl, 2-chloro-pyridin-4-yl, pyrazin-2-yl, quinolin-5-yl, quinolin-8-yl, 4-hydroxy-biphenyl-3-yl, or R2 and R3 are fused to each other, forming 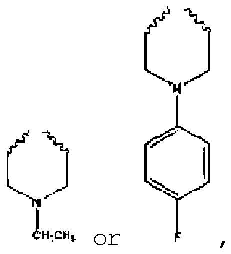 n is an integer of 0, 1 to 4,
m is an integer of 0, 1 or 2,
X represents H, linear or branched alkyl of C1∼C4, aryl of C6∼C20, hydroxy, or a halogen atom, and
Y represents H or hydroxy.

6. A method for preparing the compound of claim 1, as illustrated in the following Reaction Scheme 3, comprising mixing an amine compound of Chemical Formula 5 with sodium hydrogen carbonate in an organic solvent by stirring, and reacting the mixture with phosgene and an amine compound of Chemical Formula 6 to afford a compound of Chemical Formula 1-3: 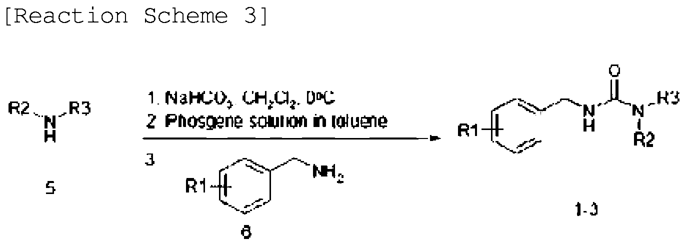 wherein,
R1 represents H, a halogen atom, linear or branched alkyl of C1∼C4, phenyl, phenyl substituted with linear or branched alkyl of C1∼C4, linear or branched alkoxy of C1∼C4, aryloxy of C6∼C20, or 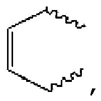 R2 and R3, which may be the same or different, independently represent H, linear or branched alkyl of C1∼C4, cycloalkyl of C3∼C8, -(linear or branched alkyl of C2∼C6)-N-(alkyl of C1∼C2)₂, -(linear or branched alkyl of C1∼C4)-NH-(linear or branched alkyl of C1∼C4), 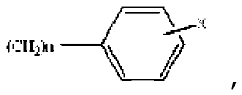 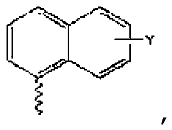 5,6,7,8-tetrahydronaphthalen-1-yl, (naphthalen-2-yl)-methyl, 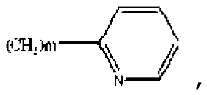 (pyridin-3-yl)-methyl, 2-chloro-pyridin-4-yl, pyrazin-2-yl, quinolin-5-yl, quinolin-8-yl, 4-hydroxy-biphenyl-3-yl, or R2 and R3 are fused to each other, forming 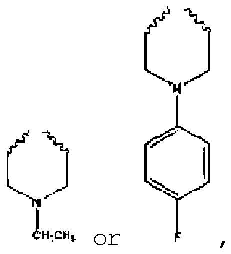 n is an integer of 0, or 1 to 4,
m is an integer of 0, 1 or 2,
X represents H, linear or branched alkyl of C1∼C4, aryl of C6∼C20, hydroxy, or a halogen atom, and
Y represents H or hydroxy.

7. A method for preparing the compound of claim 1, as illustrated in the following Reaction Scheme 4, comprising:
1) reacting a carboxylic acid compound of Chemical Formula 2 with 1,1'-carbonyldiimidazole in an organic solvent to synthesize a compound of Chemical Formula 7, and
2) mixing an amine compound of Chemical Formula 5 with sodium hydrogen carbonate in an organic solvent by stirring, and then reacting the mixture with phosgene and the compound of Chemical Formula 7, synthesized in 1), to afford a compound of Chemical Formula 1-4: 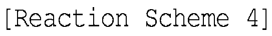 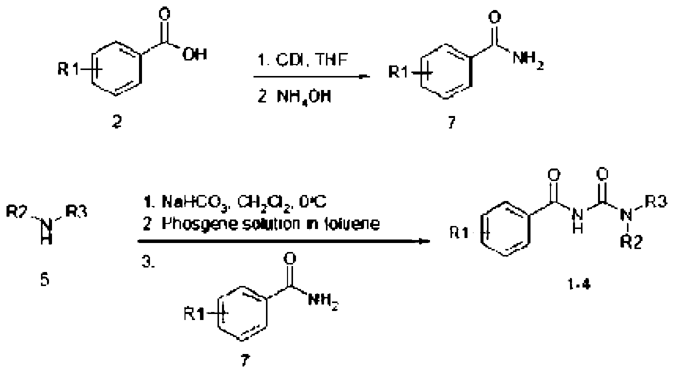 wherein,
R1 represents H, a halogen atom, linear or branched alkyl of C1∼C4, phenyl, phenyl substituted with linear or branched alkyl of C1∼C4, linear or branched alkoxy of C1∼C4, aryloxy of C6∼C20, or 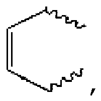 R2 and R3, which may be the same or different, independently represent, H, linear or branched alkyl of C1∼C4, cycloalkyl of C3∼C8, -(linear or branched alkyl of C2∼C6)-N-(alkyl of C1∼C2)₂, -(linear or branched alkyl of C1∼C4)-NH-(linear or branched alkyl of C1∼C4), 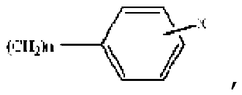 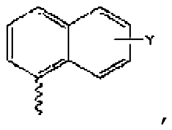 5,6,7,8-tetrahydronaphthalen-1-yl, (naphthalen-2-yl)-methyl, 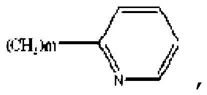 (pyridin-3-yl)-methyl, 2-chloro-pyridin-4-yl, pyrazin-2-yl, quinolin-5-yl, quinolin-8-yl, 4-hydroxy-biphenyl-3-yl, or R2 and R3 are fused to each other,
forming 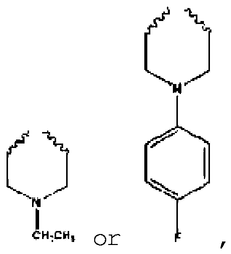 n is an integer of 0, or 1 to 4,
m is an integer of 0, 1 or 2,
X represents H, linear or branched alkyl of C1∼C4, aryl of C6∼C20, hydroxy, or a halogen atom, and
Y represents H or hydroxy, with a proviso that when R1 represents H, R2 is H, excluding that R3 represents benzyl.

8. A method for preparing the compound of claim 1, as illustrated in the following Reaction Scheme 5, comprising:
1) reacting a carboxylic acid compound of Chemical Formula 2 with oxyalyl chloride in an organic solvent to synthesize a compound of Chemical Formula 3, and
2) reacting the compound of Chemical Formula 3, synthesized in 1), with tin chloride (SnCl4), sodium cyanate (NaOCN), and an amine compound of Chemical Formula 5 to afford a compound of Chemical Formula 1-4: 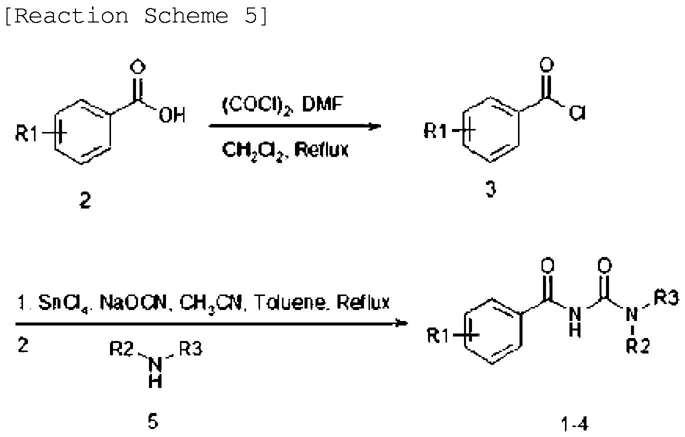 wherein,
R1 represents H, a halogen atom, linear or branched alkyl of C1∼C4, phenyl, phenyl substituted with linear or branched alkyl of C1∼C4, linear or branched alkoxy of C1∼C4, aryloxy of C6∼C20, or 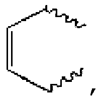 R2 and R3, which may be the same or different, independently represent, H, linear or branched alkyl of C1∼C4, cycloalkyl of C3∼C8, -(linear or branched alkyl of C2∼C6)-N-(alkyl of C1∼C2)2, -(linear or branched alkyl of C1∼C4)-NH-(linear or branched alkyl of C1∼C4), 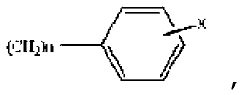 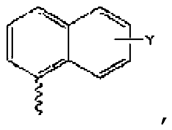 5,6,7,8-tetrahydronaphthalen-1-yl, (naphthalen-2-yl)-methyl, 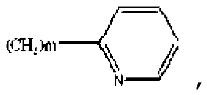 (pyridin-3-yl)-methyl, 2-chloro-pyridin-4-yl, pyrazin-2-yl, quinolin-5-yl, quinolin-8-yl, 4-hydroxy-biphenyl-3-yl, or R2 and R3 are fused to each other, forming 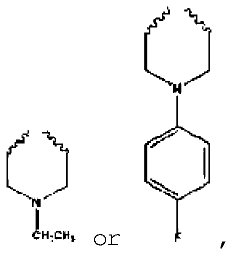 n is an integer of 0, or 1 to 4,
m is an integer of 0, 1 or 2,
X represents H, linear or branched alkyl of C1∼C4, aryl of C6∼C20, hydroxy, or a halogen atom, and
Y represent H or hydroxy, with a proviso that when R1 represents H, R2 is H, excluding that R3 represents benzyl.

9. A pharmaceutical composition for prevention or treatment of AIDS, comprising a thiourea or urea derivative of claim 1, or a derivative thereof as an active ingredient.

10. A pharmaceutical composition for prevention or treatment of AIDS, comprising as an active ingredient a compound selected from the group consisting of:
85) 4-tert-butyl-N-(4-methyl-piperazin-1-carbothioyl)-benzamide,
86) 1-benzoyl-3-(3-dimethylamino-2,2-dimethyl-propyl)-thiourea,
87) 1-benzoyl-3-(4-methyl-benzyl)-thiourea,
88) 1-benzoyl-3-(3-dimethylamino-propyl)-thiourea,
89) N-(4-ethyl-piperazin-1-carbothioyl)-benzamide,
90) 1-benzoyl-3-(4-chloro-benzyl)-thiourea,
91) 1-benzoyl-3-phenyl-thiourea,
92) 1-benzoyl-3-(2-pyridin-2-yl-ethyl)-thiourea,
93) 1-(4-tert-butyl-benzoyl)-3-phenyl-thiourea,
94) 1-(4-tert-butyl-benzoyl)-3-(pyridin-2-ylmethyl)-thiourea,
95) 1-(4-tert-butyl-benzoyl)-3-(pyridin-4-ylmethyl)-thiourea,
96) 1-(4-methyl-benzoyl)-3-(pyridin-2-yl)-thiourea,
97) 1-(4-methyl-benzoyl)-3-phenyl-thiourea,
98) 1-(4-chloro-benzoyl)-3-thiohexyl-thiourea,
99) 1-(4-chloro-benzoyl)-3-(naphthalen-2-ylmethyl)-thiourea, and
100) 1-benzoyl-3-benzyl-urea.
